# FASCICULE DE BREVET EUROPEEN

(11) **EP 3 560 021 B1**
(45) Date de publication et mention de la délivrance du brevet: **27.01.2021**
(21) Numéro de dépôt: 17832514.8
(22) Date de dépôt: 19.12.2017
(51) Int. Cl.: H01M 8/16, H01M 4/90

(54) **BIO-ÉLECTRODE POUR LA DÉTECTION ET/OU L'OXYDATION DU GLUCOSE ET SON PROCÉDÉ DE FABRICATION ET DISPOSITIF LA COMPRENANT**
BIOELEKTRODE ZUM NACHWEIS UND/ODER ZUR OXIDATION VON GLUCOSE SOWIE VERFAHREN ZU DEREN HERSTELLUNG UND VORRICHTUNG DAMIT
BIOELECTRODE FOR DETECTING AND/OR OXIDISING GLUCOSE AND METHOD FOR THE PRODUCTION THEREOF AND DEVICE COMPRISING SAME

(30) Priorité: 21.12.2016 FR 1662997
(43) Date de publication de la demande: 30.10.2019
(73) Titulaire: Centre National de la Recherche Scientifique, 75016 Paris (FR); Université Grenoble Alpes, 38400 Saint-Martin-d'Hères (FR)
(72) Inventeur: GIROUD, Fabien, 38000 Grenoble (FR); GROSS, Andrew, 38000 Grenoble (FR); COSNIER, Serge, 38920 Crolles (FR)
(74) Mandataire: LLR
(86) Numéro de dépôt international: PCT/FR2017/053689
(87) Numéro de publication internationale: WO 2018/115710

(56) Documents cités:
- US-A1- 2009 084 678
- US-A1- 2014 262 830
- DEBY FAPYANE ET AL: "High performance enzyme fuel cells using a genetically expressed FAD-dependent glucose dehydrogenase [alpha]-subunit of Burkholderia cepacia immobilized in a carbon nanotube electrode for low glucose conditions", PHYSICAL CHEMISTRY CHEMICAL PHYSICS., vol. 15, no. 24, 7 mai 2013 (2013-05-07), pages 9508-9508, XP055379514, GB ISSN: 1463-9076, DOI: 10.1039/c3cp51864g

## Description

L'invention se rapporte à une bioélectrode à base de Flavine Adénine Dinucléotide-Glucose Déshydrogènase, à ses utilisations pour la détection ou l'oxydation du glucose, à des dispositifs la comprenant, en particulier un dispositif de détection du glucose ou une pile enzymatique à biocarburant pour l'oxydation du glucose.

Elle se rapporte également à des procédés de fabrication de cette bioélectrode.

La détection du glucose dans l'urine ou le sang est importante pour détecter les personnes diabétiques.

Ainsi, un dispositif de détection du glucose est également important.

Un dispositif de contrôle du glucose qui mesure le taux de glucose dans le sang ou l'urine des patients diabétiques est une nécessité pour ces patients pour contrôler leur diabète.

De tels dispositifs doivent idéalement être des dispositifs facilement transportables, c'est-à-dire de petite taille et de faible poids.

Un grand nombre de biocapteurs ampérométriques pour la détection et la quantification du glucose ont été développés dans les dernières années.

Par ailleurs, le développement rapide de la technologie médicale a récemment permis de directement contrôler et diagnostiquer les performances d'un organe dans le corps par des dispositifs implantés.

L'alimentation en énergie de ces dispositifs par des piles à biocombustible enzymatiques est très prometteuse, en particulier lorsque le biocarburant est du glucose car dans ce cas, la pile est constamment alimentée en énergie électrique à partir du glucose contenu dans les fluides physiologiques humains.

Les détecteurs de glucose, tout comme les biopiles enzymatiques fournissant de l'énergie par oxydation du glucose, utilisent des enzymes pour agir en tant que catalyseurs convertissant le glucose en énergie électrique.

En tant qu'enzymes pour détecter le glucose et/ou l'oxyder, les enzymes Flavine Adénine Dinucléotide-Glucose Déhydrogènase (FAD-GDH) ont été particulièrement étudiées et promues dans les dernières années.

La FAD-GDH est une enzyme thermostable qui présente une activité d'oxydation hautement sélective du glucose et qui n'utilise pas l'oxygène comme accepteur d'électrons. Zafar e tal., dans "Characterization of different FAD-dependent glucose dehydrogenases for possible use in glucose-based biosensors and biofuelcells", Anal Bioanal Chem (2012) 402: 2069-2077 décrivent quant à eux une électrode constituée d'un barreau de graphite sur le sommet duquel différentes enzymes FAD-GDH ont été déposées puis réticulées dans un polymère Os. Deby Fapyane et al. dans «High performance enzyme fuel cells using a genetically expressed FAD-dependent glucose dehydrogenase α-subunit of Burkholderia cepacia immobilized in a carbon nanotube electrode for low glucose conditions », Physical Chemistry Chemical Physics 15(24), mai 2013, pp 9508-9512, XP55379514A, décrivent une électrode comprenant de la FAD-GDH couplée à de la ménadione et immobilisée sur un support de nanotubes de carbone et de Nafion®. Cependant, de telles électrodes ne permettaient pas d'obtenir une densité de courant élevée montrant ainsi que le contact électrique entre l'enzyme et le graphite n'était pas suffisant.

L'invention vise à pallier les problèmes des électrodes à base de FAD-GDH de l'art antérieur. A cet effet, l'invention propose une bioélectrode caractérisée en ce qu'elle comprend la superposition de :
- une première couche de nanotubes de carbone,
- une seconde couche, déposée sur la première couche de nanotubes de carbone, constituée de molécules aromatiques choisies dans le groupe formé par la 9,10-phénanthrènequinone, la 1,10-phénanthroline-5,6-dione, la 9,10-anthraquinone, la phénanthrène, la 1,10-phénanthroline, la 5-méthyl-1,10-phénanthroline, le pyrène, le 1-aminopyrène, l'acide pyrène-1-butyrique, et les mélanges de deux ou plus de ceux-ci,
- une troisième couche d'enzymes Flavine Adénine Dinucléotide-Glucose Déhydrogènase (FAD-GDH) adsorbée sur la seconde couche des molécules aromatiques.

La bioélectrode de l'invention peut de plus comprendre une quatrième couche en un matériau poreux et/ou hydrophile recouvrant au moins la surface libre de la troisième couche d'enzymes FAD-GDH. Ce matériau poreux et/ou hydrophile est de préférence en alginate, en chitosan, en Nafion® ou en gel de silice.

De préférence, dans la bioélectrode selon l'invention, la seconde couche demolécules aromatiques est constituée de molécules de 9,10-phénanthrènequinone ou de 1,10-phénanthroline-5,6-dione.

Egalement de préférence, dans la bioélectrode selon l'invention la couche de nanotubes de carbone comprend entre 2,5 µg et 510 µg de nanotubes de carbone par cm² de surface de couche, de préférence entre 2,5 et 100 µg de nanotubes de carbone par cm² de surface de la première couche de nanotubes de carbone.

Dans un premier mode de réalisation, la bioélectrode de l'invention comprend de plus un support en un matériau conducteur d'électrons, de préférence en carbone vitreux, sous la première couche de nanotubes de carbone.

Encore de préférence, la bioélectrode de l'invention comprend de plus un fil en un matériau électriquement conducteur relié à la surface de la couche de nanotubes de carbone qui n'est pas recouverte de la seconde couche de molécules aromatiques .

En tant que matériau électriquement conducteur, sont habituellement utilisés le cuivre, le cuivre argenté, ou le platine.

Dans la bioélectrode de l'invention, de préférence le fil conducteur est en cuivre argenté.

Dans un second mode de réalisation de la bioélectrode de l'invention, la bioélectrode de l'invention comprend de plus une couche d'enrobage imperméable aux fluides, de préférence en un polymère à base de silicone, déposée sur les surfaces libres des couches de nanotubes de carbone et d'enzymes et sur les bords de la superposition des première, seconde et troisième couches de nanotubes de carbone, de molécules aromatiques et d'enzymes FAD-GDH.

L'invention propose également un dispositif comprenant une bioélectrode selon l'invention.

Ce dispositif peut être une cellule enzymatique à biocarburant.

Ce dispositif peut également être un détecteur de glucose, lorsque la couche d'enrobage n'est pas présente.

L'invention propose encore l'utilisation d'une bioélectrode selon l'invention ou d'un dispositif selon l'invention pour l'oxydation du glucose.

L'invention propose aussi un premier procédé de fabrication d'une bioélectrode selon l'invention, caractérisé en ce qu'il comprend les étapes suivantes :
a) préparation d'une suspension ayant une concentration comprise entre 0,5 mg.mL⁻¹ à 10 mg.mL⁻¹, et de préférence 2,5 mg.mL⁻¹ de nanotubes de carbone dans un solvant organique, de préférence choisi parmi la 1-méthyl-2-pyrrolidinone (NMP), le dichlorométhane (DCM), l'acétonitrile (ACN), le 1,3-dioxolane (DXL), le diméthylformamide (DMF)), et les mélanges de deux ou plus de ceux-ci, de préférence la NMP,
b) dépôt d'une goutte (drop-casting) de la suspension de nanotubes de carbone obtenue à l'étape a) sur un support en un matériau conducteur d'électrons, de préférence en carbone vitreux,
c) évaporation du solvant organique de la suspension de nanotubes de carbone, ce par quoi une couche de nanotubes de carbone est formée sur le support,
d) préparation d'une solution comprenant des molécules aromatiques choisies dans le groupe consistant en la 9,10-phénanthrènequinone, la 1,10-phénanthroline-5,6-dione, la 9,10-anthraquinone, la phénanthrène, la 1,10-phénanthroline, la 5-méthyl-1,10-phénanthroline, le pyrène, le 1-aminopyrène, l'acide pyrène-1-butyrique, et les mélanges de deux ou plus de celles-ci, et ayant une concentration en molécules aromatiques comprise entre 0,5 mmol.L⁻¹ à 15 mmol.L⁻¹, et de préférence 2,5 mmol.L⁻¹, dissoutes dans un solvant organique choisi parmi la NMP, le DCM, l'ACN, le DXL, le DMF, et les mélanges de deux ou plus de ceux-ci, de préférence l'ACN, identique ou différent du solvant organique de la suspension de nanotubes de carbone,
e) dépôt sur une surface de la couche de nanotubes de carbone obtenue à l'étape c), de la solution de molécules aromatiques obtenue à l'étape d), par dépôt d'une goutte (drop-casting) de la solution de molécules aromatiques sur la surface de la couche de nanotubes de carbone ou par incubation d'au moins la surface libre de la couche de nanotubes de carbone dans la solution de molécules aromatiques obtenue à l'étape d), la goutte ou la solution d'incubation ayant de préférence un volume compris entre 5 et 200 µL, plus préférablement le volume étant de 175 µL,
f) évaporation du solvant de la solution de molécules aromatiques déposée à l'étape e), ce par quoi une couche de molécules aromatiques est formée sur la couche de nanotubes de carbone,
g) préparation d'une solution aqueuse d'enzymes FAD-GDH ayant de préférence une concentration en enzymes FAD-GDH comprise entre 0,1 mg.mL⁻¹ à 5 mg.mL⁻¹, et de préférence 0,75 mg.mL⁻¹ d'enzymes FAD-GDH,
h) dépôt sur la couche de molécules aromatiques obtenues à l'étape f), de la solution d'enzymes FAD-GDH obtenue à l'étape g), par incubation d'au moins la surface libre de la couche de molécules aromatiques obtenue à l'étape f), dans une solution aqueuse contenant les enzymes FAD-GDH obtenue à l'étape g), ou par dépôt d'une goutte (drop-casting) de ladite solution aqueuse contenant les enzymes FAD-GDH obtenue à l'étape g) sur la surface libre de la couche de molécules aromatiques. Le volume utilisé de solution d'enzymes pour l'incubation ou de la goutte de solution d'enzymes est de préférence compris entre 5 et 200 µL, plus préférablement de 40 µL,

Dans ce premier procédé, de préférence l'étape a) de dépôt d'une goutte de la dispersion de nanotubes de carbone est répétée au moins une fois.

L'invention propose encore un second procédé de fabrication d'une bioélectrode selon l'invention caractérisé en ce qu'il comprend les étapes suivantes :
A) préparation d'un volume de 150 mL d'une suspension de nanotubes de carbone dans un solvant organique de préférence choisi parmi la NMP, le DCM, l'ACN, le DXL, le DMF, et les mélanges de deux ou plus de ceux-ci, de préférence le DMF, la suspension ayant une concentration en nanotubes de carbone comprise entre 1 mg.mL⁻¹ et 15 mg.mL⁻¹, et de préférence de 1 mg.mL⁻¹,
B) préparation d'une solution de 5 mL contenant 0,3 mmol de molécules aromatiques choisies dans le groupe constitué par la 9,10-phénanthrènequinone, la 1,10-phénanthroline-5,6 dione, la 9,10-anthraquinone, la phénanthrène, la 1,10-phénanthroline, la 5-méthyl-1,10-phénanthroline, le pyrène, le 1-aminopyrène, l'acide pyrène-1-butyrique, et les mélanges de deux ou plus de celles-ci, de préférence de 1,10-phénanthroline-5,6 dione, dans un solvant organique identique ou différent du solvant organique de la suspension de nanotubes de carbone obtenue à l'étape a), de préférence du DMF,
C) ajout des 5 mL de la solution de molécules aromatiques obtenue à l'étape B) dans les 150 mL de la suspension de nanotubes obtenue à l'étape A), et mélange la suspension obtenue,
D) filtration de la dispersion obtenue à l'étape C) à travers un support filtrant,
E) évaporation du solvant de la suspension de nanotubes de carbones et du solvant de la solution de molécules aromatiques, ce par quoi une couche de nanotubes de carbone, elle-même recouverte sur au moins une de ses surfaces d'une couche desdites molécules aromatiques sont formées,
F) préparation d'une solution aqueuse d'enzymes FAD-GDH ayant une concentration en enzymes FAD-GDH comprise entre 0,1 et 5 mg.mL⁻¹, de préférence 0,75 mg.mL⁻¹,
G) dépôt par drop-casting de 150 µL de la solution d'enzymes FAD-GDH préparée à l'étape F) sur la surface libre de la couche de molécules aromatiques formant ainsi une couche d'enzymes FAD-GDH adsorbés sur la couche de molécules aromatiques,
H) retrait de l'assemblage obtenu à l'étape G) du support filtrant,

L'invention propose encore un procédé de fabrication d'une bioélectrode selon l'invention, caractérisé en ce qu'il comprend les étapes suivantes :
a1) préparation d'un volume de 150 mL d'une suspension de nanotubes de carbone dans un solvant organique choisi parmi la NMP, le DCM, l'ACN, le DXL, le DMF, et les mélanges de deux ou plus de ceux-ci, de préférence du DMF, la suspension ayant une concentration en nanotubes de carbone comprise entre 1 mg.mL⁻¹ et 15 mg.mL⁻¹, de préférence de 1 mg.mL⁻¹,
b1) préparation d'une solution de molécules aromatiques de concentration comprises entre 0,1 mmol.L⁻¹ et 10 mmol.L⁻¹, de préférence 10 mmol.L⁻¹ et choisies dans le groupe constitué par la 9,10-phénanthrènequinone, la 1,10-phénanthroline-5,6-dione, 9,10-anthraquinone, la phénanthrène, la 1,10-phénanthroline, la 5-méthyl-1,10-phénanthroline, le pyrène, le 1-aminopyrène, l'acide pyrène-1-butyrique, et les mélanges de deux ou plus de celles-ci, de préférence de 1,10-phénanthroline-5,6 dione, dans un solvant organique choisi parmi la NMP, le DCM, l'ACN, le DXL, le DMF, et les mélanges de deux ou plus de ceux-ci, identique ou différent du solvant organique de la suspension de nanotubes de carbone obtenue à l'étape a1), de préférence du DMF,
c1) filtration de la dispersion obtenue à l'étape a1) à travers un support filtrant,
d1) évaporation du solvant de la dispersion de nanotubes de carbone déposée à l'étape c1) ce par quoi une couche de nanotubes de carbone est formée,
e1) retrait de la couche de nanotubes de carbone obtenue à l'étape d1) du support filtrant,
f1) dépôt par drop-casting de 150 µL de la solution de molécules aromatiques obtenue à l'étape b1) sur au moins une surface de la couche de nanotubes de carbone obtenue à l'étape e1), ou par trempage de la couche de nanotubes de carbone obtenue à l'étape e1) dans la solution préparée en b1).
g1) évaporation du solvant de la solution de molécules aromatiques déposée à l'étape f1), ce par quoi une couche desdites molécules aromatiques est formée sur la couche de nanotubes de carbone,
h1) préparation d'une solution aqueuse d'enzymes FAD-GDH ayant une concentration en enzymes FAD-GDH comprise entre 0,1 et 5 mg.mL⁻¹,
il) dépôt par drop-casting de 150 µL de la solution ou par trempage de la couche de nanotubes de carbone obtenue à l'étape g1) dans la solution préparée en h1) d'enzymes FAD-GDH obtenue à l'étape h1) sur la couche de molécules aromatiques, formant ainsi une couche d'enzymes FAD-GDH adsorbés sur la couche (3).

De préférence, les second et troisième procédés de l'invention comprennent de plus, une étape k1) de fixation d'un fil en un matériau électriquement conducteur, de préférence en un matériau choisi parmi le cuivre, le cuivre argenté, le platine, plus préférablement en cuivre argenté, sur la surface de la couche de nanotubes de carbone non recouverte de la couche de molécules aromatiques, cette étape étant mise en œuvre avant ou après l'étape G) ou i1) de dépôt de la couche d'enzymes FAD-GDH.

De préférence, tous les procédés de fabrication d'une bioélectrode selon l'invention comprennent de plus une étape d'enrobage de la superposition : couche de nanotubes de carbone / couche de molécules aromatiques /couche d'enzymes FAD-GDH/fil en un matériau électriquement conducteur, de préférence avec un composé à base de silicone.

L'invention sera mieux comprise et d'autres caractéristiques et avantages de celle-ci apparaîtront plus clairement à la lecture de la description explicative qui suit et qui est faite en référence aux figures dans lesquelles :
- la figure 1 montre un premier mode de réalisation de l'électrode de l'invention, décrit à l'exemple 1,
- la figure 2 montre un deuxième mode de réalisation de l'électrode de l'invention, décrit à l'exemple 2,
- la figure 3 montre un troisième mode de réalisation de l'électrode de l'invention, décrit à l'exemple 3,
- la figure 4 montre les voltampérogrammes obtenus avec les électrodes obtenues aux exemples 1, 4 à 8 et aux exemples comparatifs 2 et 4 à 6,
- la figure 5 montre les voltampérogrammes obtenus avec les électrodes obtenues à l'exemple 13.

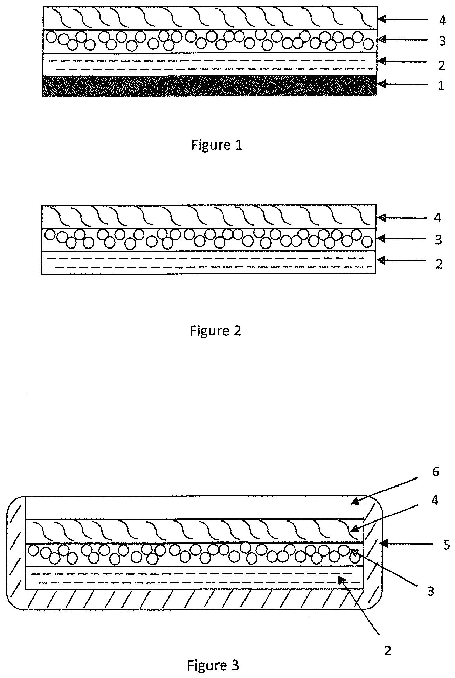

Comme montré en figure 1, dans un premier mode de réalisation, la bioélectrode de l'invention comprend la superposition sur un support conducteur, noté 1 en figure 1, d'une couche, notée 2 en figure 1, de nanotubes de carbone, sur laquelle est déposée une couche, notée 3 en figure 1, de molécules aromatiques, sur laquelle est déposée une couche, notée 4 en figure 1, d'enzymes Flavine Adénine Dinucléotide-Glucose Déhydrogènase (FAD-GDH).

Dans l'invention, par nanotube de carbone, on entend un nanotube de carbone dont au moins une dimension est inférieure à 1500 nm.

La couche 2 de nanotubes de carbone peut être constituée de nanotubes de carbone simple paroi ou multi-parois.

Cependant, de préférence, elle est constituée de nanotubes de carbone multi-parois.

En effet, les nanotubes de carbone mutli-parois ont un diamètre externe plus élevé et donc une surface développée plus élevée, ce qui permet de mieux immobiliser les molécules aromatiques et les enzymes.

De préférence, les nanotubes de carbone ont un rapport longueur (L) sur diamètre noté L/diamètre compris entre 100 et 5000.

De préférence les nanotubes de carbone ont une longueur de 1,5 µm et un diamètre interne de 9,5 nm.

La couche 2 de nanotubes de carbone doit être poreuse.

La taille des pores dans la couche 2 de nanotubes de carbone doit être supérieure à 8 nm, la taille de l'enzyme FAD-GDH étant de 78 Å (7,8 nm).

De préférence, la taille des pores de la couche 2 de nanotubes de carbone est comprise entre 8 et 50 nm.

Plus préférablement, la taille des pores de la couche de nanotubes de carbone est de 10 nm.

Les molécules aromatiques formant la couche 3 sont liées par interaction π-π sur la couche 2 de nanotubes de carbone.

Bien qu'en figure 1 (comme en figures 2 et 3), la couche 2 de nanotubes de carbone ainsi que la couche 3 de molécules aromatiques sont représentées comme une véritable superposition, l'homme de l'art comprendra aisément que la couche 2 de nanotubes de carbone étant poreuse est constituée elle-même de plusieurs "sous-couches" de nanotubes de carbone, les molécules aromatiques se lient également aux nanotubes de carbone (sous-couches).

Ainsi, les couches 2 et 3 ne sont pas individualisées mais imbriquées l'une dans l'autre.

Les molécules aromatiques favorisent le transfert des électrons entre l'enzyme et les nanotubes de carbone.

Elles jouent de plus le rôle de molécules orientant la structure tridimensionnelle de l'enzyme qui sera ensuite adsorbée sur ces molécules de telle sorte à favoriser le transfert électronique de l'enzyme vers les nanotubes de carbone.

Les molécules aromatiques formant la couche 3 sont choisies parmi la 9,10-phénanthrènequinone, la 1,10-phénanthroline-5,6-dione, la 9,10-anthraquinone, la phénanthrène, la 1,10-phénanthroline, la 5-méthyl-1,10-phénanthroline, le pyrène, le 1-aminopyrène, l'acide pyrène-1-butyrique, et les mélanges de deux ou plus de ces molécules.

De préférence, les molécules aromatiques formant la couche 3 sont des molécules de phénanthrène ou de 1,10-phénanthroline-5,6-dione.

Et il est particulièrement surprenant que de telles molécules aromatiques puissent jouer le rôle de médiateur entre les nanotubes de carbone et l'enzyme FAD-GDH car, en particulier, la molécule de 1,10-phénanthroline est connue pour être un inhibiteur de l'enzyme FAD-GDH, pour l'oxydation du glucose car elle prend la place du glucose dans cette réaction d'oxydation.

L'activité inhibitrice de la 1,10-phénanthroline pour la réaction de la FAD-GDH est d'ailleurs soulignée par les sociétés commercialisant cette enzyme, voir par exemple Sekisui Enzymes, "Glucose Dehydrogenase FAD Dependent, Catalogue n°GLDE-70-1192, E.C number 1.1.99.10".

Cette activité inhibitrice, pour les molécules 9,10-phénanthrènequinone, 1,10-phénanthroline-5,6-dione, phénanthrène et 1,10-phénanthroline, est confirmée par des essais en voltampérométrie cyclique.

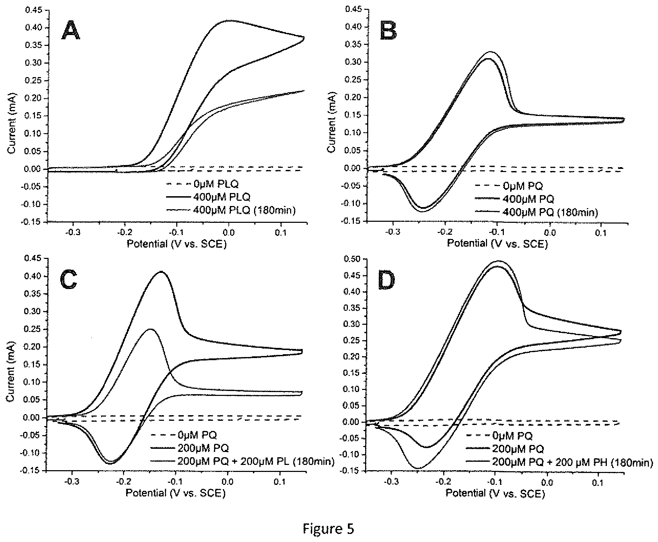

Les voltampérogrammes obtenus lors de ces essais sont montrés en figure 5. Ils ont été obtenus avec des électrodes dont la couche d'enzyme FAD-GDH est directement déposée sur la couche 2 de nanotubes de carbone en présence de 100 mmol L⁻¹ de glucose en solution.

Les molécules aromatiques 9,10-phénanthrènequinone, 1,10-phénanthroline-5,6-dione, phénanthrène et 1,10-phénanthroline sont ensuite progressivement ajoutées en solution.

La présence de 200 µmol L⁻¹ de 9,10-phénanthrènequinone ou de phénanthrène n'engendrent pas de diminution drastique des courants catalytiques mesurés pour des durées d'échantillonnage inférieur à 3 heures.

En revanche, la présence de molécule aromatique à base de pyridines telles que la 1,10-phénanthroline-5,6-dione et la 1,10-phénanthroline engendre une progressive diminution des performances de la bioélectrocatalyse dans le temps.

La couche d'enzymes FAD-GDH, notée 4 dans les figures 1 à 3, est une couche de fonctionnalisation des molécules aromatiques de la couche notée 3 dans les figures 1 à 3.

L'enzyme ou les enzymes formant la couche 4 est (sont) adsorbée(s) sur les molécules aromatiques formant la couche 3.

Là encore, bien qu'en figure 1 (tout comme en figures 2 et 3), la couche 4 d'enzymes FAD-GDH est représentée comme une couche distincte en contacte uniquement avec la couche 3 de molécules aromatiques, l'homme de l'art comprendra aisément que les enzymes FAD-GDH constituant la couche 4 sont également adsorbés sur les molécules organiques qui sont elles-mêmes liées (par liaison π-π) aux nanotubes de carbone présents dans les sous-couches de la couche 2 de nanotubes de carbone.

Autrement dit, la superposition des couches 2, 3 et 4 est constituée en réalité d'assemblages individuels nanotube de carbone-molécule aromatique-enzyme FAD-GDH.

Dans ce premier mode de réalisation, de préférence, la quantité de nanotubes de carbone déposée sur la surface du support 1 est comprise entre 2,5 µg de nanotubes de carbone par cm² de surface du substrat 1 et 510 µg de nanotubes de carbone par cm² de surface du support 1, pour une application de l'électrode dans un capteur de glucose et est comprise entre 5 µg et 100 µg de nanotubes de carbone par cm² de surface du support 1 lorsque l'électrode est destinée à être utilisée en tant qu'électrode d'une biopile à biocarburant pour l'oxydation du glucose.

En effet, dans une telle application, si la quantité de nanotubes de carbone par cm² de surface du support est inférieure à 5 µg, il n'y a plus assez de nanotubes de carbone pour produire un courant catalytique qui permette à la bio-pile de fournir de l'énergie électrique suffisante et lorsque la quantité de nanotubes de carbone est supérieure à 100 µg par cm² de surface du support 1, la couche 2 de nanotubes de carbone est formée de plusieurs sous couches de nanotubes de carbone empilées les unes sur les autres et les couches internes (celles qui ne sont pas à la surface) de nanotubes de carbone ne voient une concentration plus faible en glucose puisque celui-ci est oxydé principalement dans les couches supérieures.

Dans ce premier mode de réalisation de l'invention l'électrode est une électrode supportée sur un support noté 1 en figure 1.

Mais, dans un second mode de réalisation montré en figure 2, la bioélectrode de l'invention est auto-supportée.

La superposition de couches 2, 3 et 4 telle que décrite dans le premier mode de réalisation est alors réalisée sur une surface de ce support 1.

Le support 1 est en un matériau électroniquement conducteur, de préférence en carbone vitreux.

Dans le second mode de réalisation de la bioélectrode de l'invention, celle-ci comprend de plus un fil conducteur en un matériau électriquement conducteur pour transférer le courant de l'électrode vers le dispositif de détection ou d'oxydation du glucose.

Dans tous les modes de réalisation de la bioélectrode de l'invention, et comme montré en figure 3, au moins la surface libre de la couche 4 d'enzymes FAD-GDH est protégée par une couche notée 6, en figure 3, en un matériau poreux et/ou hydrophile.

Cette couche 6 a un rôle de protection des enzymes FAD-GDH en empêchant le déplacement des enzymes FAD-GDH dans le milieu dans lequel la bioélectrode de l'invention est destiné à être utilisée.

Cette couche 6 doit être en un matériau poreux pour permettre l'accès du fluide contenant le glucose aux enzymes FAD-GDH et au déplacement des produits de la réaction d'oxydation (le gluconolactone et les protons), tout en ne laissant pas passer les enzymes FAD-GDH.

Dès lors, le diamètre des pores du matériau constituant la couche 6 doit être inférieur à 78 Å. Il est de préférence inférieur à 75 Å. Cela permet de d'encapsuler l'enzyme sur l'électrode tout en assurant la diffusion du substrat et du produit jusqu'à et hors de l'électrode respectivement.

Le matériau constituant la couche 6 est de préférence hydrophile, également, pour améliorer le déplacement, dans la bioélectrode, du milieu aqueux dans laquelle elle sera placée.

De préférence, la couche 6 est en alginate, ou en chitosan, ou en Nafion® ou en gel de silice.

La couche 6 recouvre de préférence toute la bioélectrode.

Cependant, lorsqu'une couche 5 d'enrobage en un matériau imperméable aux fluides est fournie, seule la surface de la couche 4 doit impérativement être recouverte avec la couche 6.

En effet, lorsque la bioélectrode de l'invention est destinée à être placée dans une biopile pour l'oxydation du glucose, elle est de préférence enrobée, comme montré en figure 3, dans une couche d'un matériau imperméable aux fluides formant ainsi une couche d'enrobage, notée 5 en figure 3.

Cette couche d'enrobage 5 ne doit pas recouvrir la couche 6.

La couche 5 est en un matériau imperméable aux fluides, de préférence en un matériau à base de silicone.

Cette couche 5 permet d'assurer la stabilité de la superposition de couches constituant la bioélectrode de l'invention et d'empêcher le déplacement des nanotubes de carbone, des molécules aromatiques et des enzymes FAD-GDH dans lequel la bioélectrode de l'invention est à placer.

Cette couche 5 est particulièrement importante, lorsque la couche 6 ne recouvre pas l'ensemble de la superposition des couches 2, 3 et 4 et éventuellement du support 1.

L'invention propose également un dispositif comprenant une bioélectrode selon l'invention.

Ce dispositif peut être une biopile à biocarburant dans laquelle l'énergie est produite par l'oxydation du glucose.

En effet, la bioélectrode de l'invention est de très petite taille et très légère et peut être placée dans un dispositif portable miniaturisé ou implanté directement dans le corps d'un humain ou d'un animal.

Ce dispositif peut également être un dispositif de détection et de quantification du glucose dans un fluide corporel humain tel que le sang, l'urine, les larmes ou la sueur. Dans ce cas, la bioélectrode n'est pas enrobée dans la couche 5 mais recouverte en totalité de la couche 6.

La bioélectrode de l'invention peut être fabriquée par des procédés très simples qui ne mettent en œuvre aucune réaction chimique ni étape de synthèse de création de polymères actifs redox ou d'assemblages supra-moléculaires compliqués.

De plus, avec les procédés de l'invention, les épaisseurs des bioélectrodes et les quantités de molécules aromatiques méadiatrices et d'enzymes peuvent être modifiées selon la sensibilité et l'efficacité requise pour l'application voulue de ces bioélectrodes.

Un premier procédé de fabrication de la bioélectrode selon l'invention est un procédé de fabrication d'une bioélectrode supportée sur un support 1 rigide.

Le support 1 est en un matériau électriquement conducteur, de préférence en carbone vitreux.

Ce procédé comprend, comme tous les autres procédés selon l'invention : une étape de préparation d'une suspension de nanotubes de carbone dans un solvant organique.

A titre de solvant organique utilisable pour cette suspension, on citera la NMP, le DCM, l'ACN, le DXL le DMF et les mélanges de deux ou plus de ceux-ci.

Le solvant organique préféré est la NMP car ce solvant a une meilleure affinité (mouillabilité) avec le support 1, lorsque ce dernier est en carbone vitreux.

La suspension devra avoir une concentration en nanotubes de carbone permettant de déposer, sous la forme d'une goutte (méthode dite "drop-casting" en anglais) une quantité de nanotubes de carbone comprise entre 2,5 µg et 510 µg par cm² de surface du support 1, lorsque la bioélectrode de l'invention est à utiliser dans un capteur de glucose.

Généralement une concentration comprise entre 0,5 mg.mL⁻¹ et 10 mg.mL⁻¹ est utilisée. Si la concentration en nanotubes de carbone de la dispersion est trop élevée, les couches de nanotubes de carbone obtenues sont moins stables et moins reproductibles en raison d'une dispersion moins bonne dans la solution.

De préférence une concentration de 2,5 mg.mL⁻¹ est utilisée.

La quantité de nanotubes déposés sur le support 1 est de préférence comprise entre 5 µg et 100 µg de nanotubes de carbone par cm² de surface du support lorsque la bioélectrode est destinée à être utilisée pour fabriquer une biopile à combustible biologique.

En effet, dans ce cas la quantité de nanotubes de carbone est inférieure à 5 µg par cm² de surface du support, il n'y a plus assez de nanotubes de carbone pour fournir un courant catalytique qui permette à la pile de fournir de l'énergie.

Mettre plus de 100 µg par cm² de surface du support, l'épaisseur de cette couche est alors importante et les nanotubes de carbone qui sont sous les autres nanotubes de carbone ne 'servent' pas pour l'oxydation du glucose car le glucose présent dans le fluide est oxydé par les nanotubes plus en surface du support ; le glucose ne diffuse donc plus dans toute la couche formée par les nanotubes.

Ensuite, toujours comme pour tous les procédés de l'invention, ce premier procédé de l'invention comprend une étape de préparation d'une solution de la ou des molécules aromatiques voulues par dissolution de cette (ces) molécule(s) aromatique(s) dans un solvant organique. Le solvant peut être identique ou différent du solvant organique de la suspension de nanotubes de carbone.

Ainsi, à titre de solvant organique utilisable pour solubiliser la ou les molécules organiques, on peut citer la NMP, le DCM, l'ACN, le DXL, le DMF et les mélanges de deux ou plus de ceux-ci.

Cependant, de préférence, le solvant organique pour la mise en solution de la ou des molécules aromatiques est identique au solvant organique utilisé pour mettre en suspension les nanotubes de carbone.

A ce titre, il est également de préférence du NMP dans le premier procédé de l'invention.

La concentration en molécules organiques dans cette solution est de préférence comprise entre 0,5 mmol L⁻¹ à 15 mmol L⁻¹. Elle est de préférence de 2,5 mmol L⁻¹.

Le premier procédé de l'invention, comme tous les procédés de l'invention, comprend également une étape de préparation d'une solution aqueuse d'enzymes FAD-GDH.

Par solution aqueuse on entend une solution tampon de pH compris entre 4,0 et 9,0. Les solutions tampons qui peuvent être utilisées ainsi que leurs proportions dans l'eau sont à base d'acide citrique, d'acide acétique, de phosphate, de tout autre espèce susceptible de jouer le rôle d'un tampon ou de mélanges de deux ou plus de ceux-ci pour garder des pHs compris entre 4,0 et 9,0.

La concentration en enzymes FAD-GDH dans cette solution aqueuse est de préférence comprise entre 0,1 mg.mL⁻¹ et 5 mg.mL⁻¹. Elle est plus préférablement de 0,75 mg.mL⁻¹.

Une fois la suspension de nanotubes de carbone préparée, dans le premier procédé de fabrication de la bioélectrode de l'invention, une goutte d'un volume maximal de 20 µL de la dispersion de nanotubes de carbone est déposée sur une surface du support 1.

En effet, si le volume de goutte déposé est plus important, l'épaisseur de la couche de nanotubes de carbone est augmentée et pourrait être nuisible à la diffusion du glucose dans la couche poreuse.

Eventuellement cette étape peut être répétée une fois ou plus pour augmenter la quantité de nanotubes de carbone déposés et ainsi augmenter la surface spécifique active de la bioélectrode.

Une fois la formation d'une couche 2 de nanotubes de carbone de l'épaisseur voulue obtenue, la solution de molécules aromatiques est mise en contact avec cette couche de nanotubes de carbone, en général à température ambiante (entre 15 et 30°C).

Cette mise en contact peut être réalisée par incubation de l'ensemble support 1 recouvert de la couche 2 de nanotubes de carbone dans la solution de molécules aromatiques ou de seulement la surface libre de la couche 2 de nanotubes de carbone par dépôt d'une goutte de la solution de molécules aromatiques directement sur la couche de nanotubes de carbone.

La solution de molécules aromatiques est laissée en contact avec la couche 2 de nanotubes de carbone pendant une durée comprise entre 5 minutes et 1 heure pour permettre une fonctionnalisation des nanotubes de carbone.

Une durée supérieure à 1 heure ne permet pas d'obtenir l'adsorption de plus de molécules aromatiques.

Ensuite l'assemblage du support 1 / couche 2 de nanotubes de carbone / couche 3 de molécules aromatiques en solution est rincé avec de l'eau distillée pour éliminer les molécules aromatiques qui ne seraient adsorbées que faiblement sur les nanotubes de carbone de la couche 2.

En effet, les molécules aromatiques sont adsorbées sur les nanotubes de carbone par une interaction π-π et forment ainsi une couche 3 de molécules aromatiques.

Ensuite, l'assemblage support 1 / couche de nanotubes de carbone 2 / couche 3 de molécules aromatiques est mis en contact avec la solution aqueuse contenant les enzymes FAD-GDH pendant une période de temps suffisante pour l'orientation préférentielle de l'enzyme FAD-GDH face aux molécules organiques.

Généralement, une durée variant de 4 heures à 1 jour à 4°C est utilisée.

La solution aqueuse d'enzymes FAD-GDH est une solution tamponnée à un pH compris entre 4,0 et 9,0 et de préférence un pH de 7,0 pour ne pas endommager l'enzyme FAD-GDH.

Enfin, la bioélectrode ainsi obtenue est rincée abondamment avec la solution tamponnée (toujours pour ne pas endommager l'enzyme FAD-GDH) pour éliminer les enzymes adsorbées de façon lâche formant ainsi une couche 4 d'enzyme FAD-GDH.

Si la bioélectrode ainsi obtenue n'est pas utilisée immédiatement, elle est conservée dans une solution tampon ou à l'air jusqu'à utilisation ou réutilisation.

Un second procédé pour fabriquer une bioélectrode selon l'invention est un procédé dans lequel la bioélectrode est autosupportée, c'est-à-dire qu'elle ne comprend pas de support 1.

Dans ce second procédé, une fois la suspension de nanotubes de carbone et les solutions de molécules et d'enzymes FAD-GDH préparées, la solution de molécules aromatiques est ajoutée à la suspension de nanotubes de carbone et la solution obtenue est traitée aux ultrasons généralement pendant 30 minutes.

Dans le second procédé de l'invention, la suspension de nanotubes de carbone a une concentration comprise entre 1 mg.mL⁻¹ et 15 mg.mL⁻¹, et de préférence de 1 mg.mL⁻¹.

Cette dispersion mixte nanotubes de carbone-molécules aromatiques est ensuite filtrée sous vide sur un filtre en un matériau polymère chimiquement inerte.

Un tel matériau polymère chimiquement inerte est de préférence le polytétrafluoroéthylène (PTFE).

On a ainsi formé ce qui est appelé 'Buckypaper' dans l'art.

Ce 'Buckypaper' est retiré du filtre et laissé à sécher à plat, généralement pendant une nuit à température ambiante. Il est ensuite découpé à la taille voulue.

Ensuite, ce 'Buckypaper' est mis à incuber dans, ou est recouvert d'une goutte, de la solution aqueuse tamponnée des enzymes FAD-GDH pendant entre 4 heures et 1 jour, de préférence une nuit, à 4°C.

Après cette période d'incubation ou de maintien en contact, le 'Buckypaper' est rincé avec une solution tampon pour éliminer les enzymes adsorbées de façon lâche à la surface des molécules aromatiques. Les solutions tampons qui peuvent être utilisées ainsi que leurs proportions dans l'eau sont à base d'acide citrique, d'acide acétique, de phosphate, de tout autre espèce susceptible de jouer le rôle d'un tampon ou de mélanges de deux ou plus de ceux-ci pour garder des pHs compris entre 4,0 et 9,0.

Pour assurer la connexion électrique de l'électrode, un fil métallique est attaché au dos de l'électrode par exemple avec une pâte de carbone suivi par l'application d'un matériau silicone.

Cette étape d'attache du fil métallique peut être effectuée avant ou après la formation de la couche 4 d'enzymes FAD-GDH.

Le troisième procédé de l'invention consiste également à préparer un 'Buckypaper', mais cette fois uniquement constitué de nanotubes de carbone.

Ainsi, la différence entre le second procédé et le troisième procédé de l'invention est que la suspension de nanotubes de carbone n'est pas mélangée à la solution de molécules aromatiques avant d'être déposée sur un support filtrant mais est déposée seule sur le support filtrant.

Puis le solvant de la suspension de nanotubes de carbone est filtré et la couche de nanotubes de carbone ainsi obtenue est retirée du support filtrant : on a obtenu un 'Buckypaper' uniquement constitué de nanotubes de carbone.

Ensuite, la solution de molécules aromatiques est déposée par 'drop-casting' sur cette couche de nanotubes de carbone, comme dans le premier procédé de l'invention.

On procède ensuite comme dans le premier procédé de l'invention.

Afin de mieux faire comprendre l'invention, on va maintenant en décrire, à titre purement illustratif et non limitatif plusieurs exemples de mise en œuvre.

### Exemple 1 : Fabrication d'une bioélectrode selon l'invention supportée sur un support en carbone vitreux.

Cet exemple sera décrit en référence à la figure 2 annexée.

Une suspension de nanotubes de carbone multi-parois (9,5 nm de diamètre, pureté supérieure à 95%) est obtenue à partir de nanotubes de carbone non fonctionnalisés par traitement aux ultrasons dans le NMP pendant 30 minutes.

La concentration en nanotubes de carbone de cette suspension est de 2,5 mg.mL⁻¹.

La dispersion est stable et est vigoureusement secouée pour améliorer la reproductibilité du dépôt de la couche de nanotubes de carbone sur le support 1.

Une goutte d'un volume de 20 µL de cette suspension est déposée sur la surface du support 1 en carbone vitreux,

La NMP est ensuite évaporé sous vide moyen (Videₘₐₓ = 10⁻³ mbar).

On obtient ainsi le support 1 en carbone vitreux revêtu sur une de ses surfaces d'une couche 2 de nanotubes de carbone secs.

Cet assemblage est ensuite mis à incuber dans une solution de 1,10-phénanthroline-5,6-dione à une concentration de 2,5 mmol.L⁻¹, dans de l'ACN, pendant 1 heure à température ambiante.

L'assemblage ainsi obtenu est ensuite rincé abondamment avec de l'eau distillée pour éliminer les molécules de 1,10-phénanthroline-5,6-dione non complètement liés aux nanotubes de carbone de la couche 2.

On obtient alors à ce stade un assemblage support 1 / couche 2 de nanotubes de carbone / couche 3 de molécules de 1,10-phénanthroline-5,6-dione.

Une goutte de 40 µL de solution d'enzymes FAD-GDH tamponnée à pH 7,0 et ayant une concentration de 0,75 mg.mL⁻¹ d'enzymes est ensuite déposée sur la surface de l'assemblage obtenu des trois couches 1, 2 et 3.

Cet assemblage est ensuite laissé une nuit à 4°C pour adsorption des enzymes.

Finalement, l'assemblage obtenu est rincé abondamment avec une solution tampon McIlvaine (0,2 mol L⁻¹ Na₂HPO_{4,} 0,1 mol L⁻¹ acide citrique) tamponnée à pH 7,0 pour éliminer les enzymes adsorbées de façon lâche sur la surface des molécules de 1,10-phénanthroline-5,6-dione.

### Exemple 2 : Fabrication d'une bioélectrode selon l'invention auto-supportée (électrode 'Buckypaper').

Une suspension de nanotubes de carbone multi-parois dans du DMF est préparée par addition de 150 mg de nanotubes de carbone multi-parois non fonctionnalisés (9,5 nm de diamètre, pureté supérieure à 95%) dans 150 mL de DMF.

La suspension obtenue a une concentration en nanotubes de carbone de 1 mg.mL⁻¹.

La dispersion est ensuite traitée aux ultrasons pendant 30 minutes.

Une solution de molécules de 1,10-phénanthroline-5,6 dione est ensuite préparée par dissolution de 65 mg de solide dans un volume de 5 mL de DMF et est ajoutée lentement dans la suspension de nanotubes de carbone multi-parois jusqu'à une concentration finale en molécule de 1,10-phénanthroline-5,6 dione de 2 mmol L⁻¹.

La suspension résultante est alors traitée aux ultrasons pendant 30 minutes supplémentaires.

Après une agitation vigoureuse manuelle pendant une minute, 66 mL de la suspension ainsi obtenue sont filtrés à travers un filtre millipores en PTFE (JHWP 0,45 µm de taille de pores, 46 mm de diamètre) en utilisant une pompe à vide (Videₘₐₓ = 10⁻³ mbar). L'ensemble est lavé avec de l'eau distillée et laissé au repos pendant une heure.

Puis, l'assemblage couche 2 de nanotubes de carbone / couche 3 de molécules aromatiques est retiré du support de filtre et découpé en précurseur d'électrode de 10 mm de diamètre (surface spécifique géométrique de 0,785 cm²).

Un contact électrique est obtenu via un fil métallique maintenu par une pâte de carbone (Acheson ; ELECTRODAG 423 SS) sur la surface libre de la couche 2 de nanotubes de carbone de l'assemblage obtenu.

Puis 150 µL d'une solution d'enzyme FAD-GDH sont déposés sur la surface de l'assemblage de la couche 2 de nanotubes de carbone / couche 3 de molécules de 1,10-phénanthroline-5,6-dione.

La solution d'enzymes FAD-GDH est une solution à 5 mg.mL⁻¹ d'enzymes FAD-GDH préparée dans un tampon McIlvaine pH 7,0 (0,2 mol.L⁻¹ Na₂HPO₄, 0,1 mol L⁻¹ acide citrique).

L'ensemble est placé au réfrigérateur (4°C) une nuit puis rincé avec la solution tampon.

Puis, l'autre face de la bioélectrode et ses côtés sont scellés avec une pâte de silicone (fabricants : Rubson, GEB) pour former une couche d'enrobage 5.

### Exemple 3

On a procédé comme à l'exemple 2 mais on a de plus encapsulé l'ensemble dans une couche d'enrobage 6.

Cette couche peut être en alginate, chitosan, Nafïon®, ou en gel de silice, par exemple.

On a obtenu l'électrode montrée en figure 3.

### Exemple 4

On a procédé comme à l'exemple 1 mais en remplaçant la molécule aromatique 1,10-phénanthroline-5,6-dione par une molécule de pyrène.

### Exemple 5

On a procédé comme à l'exemple 1 mais les molécules aromatiques 1,10-phénanthroline-5,6 dione ont été remplacées par des molécules de 9,10-phénanthroline.

### Exemple 6

On a procédé comme à l'exemple 1 mais les molécules aromatiques 1,10-phénanthroline-5,6 dione ont été remplacées par la molécule de 5-méthyl-1,10-phénanthroline.

### Exemple 7

On a procédé comme à l'exemple 1 mais les molécules aromatiques 1,10-phénanthroline-5,6 dione ont été remplacées par la molécule de 1,4-naphthoquinone.

### Exemple 8

On a procédé comme à l'exemple 1 mais les molécules aromatiques 1,10-phénanthroline-5,6 dione ont été remplacées par la molécule de 9,10-phénanthrènequinone.

### Exemple 9

On a procédé comme à l'exemple 2 mais on a remplacé les molécules de 1,10-phénanthroline-5,6-dione par des molécules de 9,10-phénanthroline.

### Exemple 10

On a procédé comme à l'exemple 2 mais on a remplacé les molécules de 1,10-phénanthroline-5,6-dione par des molécules de 5-méthyl 1,10-phénanthroline,

### Exemple comparatif 1

Une bioélectrode de contrôle constituée uniquement du film de nanotubes de carbone a été obtenu par préparation d'une suspension de nanotubes de carbone multi-parois à une concentration de 1 mg.mL⁻¹ par addition de 150 mg de nanotubes de carbone non fonctionnalisés (9,5 nm de diamètre, pureté supérieure à 95%) dans 150 mL de DMF.

Cette suspension est ensuite secouée manuellement pendant une minute.

66 mL de la suspension est filtrée à travers un filtre millipores en PTFE (JHWP, taille de pores 0,45 µm, 46 mm de diamètre) en utilisant une pompe à vide (Videₘₐₓ = 10⁻³ mbar).

La couche de nanotubes de carbone a été lavée avec de l'eau distillée et laissée au repos pendant une heure.

Après la filtration, le 'Buckypaper' résultant a été laissé séché à température ambiante.

Puis le 'Buckypaper' obtenu est enlevé précautionneusement du filtre et coupé en une électrode de 10 mm de diamètre (surface géométrique 0,785 cm²).

### Exemple comparatif 2

Une dispersion de nanotubes de carbone multi-parois a été préparée comme à l'exemple comparatif 1 sauf que la dispersion obtenue avait une concentration de 2,5 mg.mL⁻¹.

Ainsi, la quantité de nanotubes de carbone placée dans les 2 mL de NMP était non pas de 150 mg mais de 5 mg.

Une goutte d'un volume de 20 µL de cette suspension a ensuite été déposée sur un support en carbone vitreux ayant une surface de 0,196 cm².

L'épaisseur de la couche de nanotubes de carbone obtenue était de 12 ± 4 µm.

### Exemple comparatif 3

Une bioélectrode a été préparée en suivant la procédure de l'exemple 2 mais en s'arrêtant avant l'étape de dépôt d'une goutte de la solution à 5 mg.mL⁻¹ d'enzymes FAD-GDH.

Ainsi, l'électrode obtenue n'était constituée que des nanotubes de carbone sur lesquels étaient adsorbées les molécules aromatiques 1,10-phénanthroline-5,6-dione.

### Exemple comparatif 4

On a préparé une bioélectrode comme à l'exemple 1 sauf que l'étape d'adsorption de la molécule aromatique n'a pas été mise en œuvre.

On a ainsi obtenu une bioélectrode constituée d'un support 1 en carbone vitreux recouvert sur une de ses faces d'une couche 2 en nanotubes de carbone sur laquelle sont adsorbées directement les enzymes FAD-GDH.

### Exemple comparatif 5

On a procédé comme à l'exemple 1 mais la molécule aromatique utilisée était la flavine adénine dinucléotide (FAD : cofacteur de l'enzyme FAD-GDH).

### Exemple comparatif 6

On a procédé comme à l'exemple 1 mais la molécule aromatique utilisée était l'ester N-hydroxysuccinimide de l'acide pyrène butyrique.

### Exemple comparatif 7

On a procédé comme à l'exemple 2 mais les molécules aromatiques 1,10-phénanthroline-5,6-dione ont été remplacées par des molécules de 1,4-naphthoquinone.

### Exemple 11 : Caractérisation des couches de nanotubes de carbone.

La caractérisation de la couche de nanotubes de carbone obtenue à l'exemple comparatif 1 a été effectuée avec un microscope confocal à balayage laser.

L'épaisseur du film de nanotubes de carbone obtenu à l'exemple comparatif 1 était de 275 ± 45 µm.

La couche de nanotubes de carbone obtenue à l'exemple comparatif 1 a été caractérisée avec un microscope électronique à balayage à un grossissement de 40 000.

La caractérisation de la couche de nanotubes de carbone obtenue à l'exemple comparatif 2 a été effectuée avec un microscope confocal à balayage laser.

L'épaisseur du film de nanotubes de carbone obtenu à l'exemple comparatif 2 était de 12 ± 4 µm.

La couche de nanotubes de carbone obtenue à l'exemple comparatif 2 a été caractérisée avec un microscope électronique à balayage à un grossissement de 40 000.

On a constaté que la couche de nanotubes de carbone obtenue à l'exemple comparatif 1 était plus dense que la couche de nanotubes de carbone obtenue à l'exemple comparatif 2, ce qui signifie que la filtration permet d'obtenir des couches plus compactes de nanotubes de carbone.

L'électrode obtenue à l'exemple comparatif 7, c'est-à-dire un 'Buckypaper' constitué de nanotubes de carbone sur lesquels les molécules aromatique médiatrices 1,10-phénanthroline-5,6-dione sont adsorbées avant la filtration du mélange nanotubes / molécule aromatique, a également été étudiée au microscope électronique à balayage à un grossissement de 40 000.

Cette étude a montré que la molécule aromatique 1,10-phénanthroline-5,6-dione augmente encore l'agglutination des tubes entre eux, comme attendu, la 1,10-phénanthroline-5,6-dione jouant le rôle d'un liant entre les nanotubes de carbone en échangeant des électrons π.

Il en résulte donc des 'Buckypaper' plus solides et plus flexibles que les 'Buckypaper' non fonctionnalisés donnés en exemple comparatif 1 qui sont eux bien plus fragiles.

**Exemple 12 :** Caractérisation électrochimique des électrodes obtenues aux exemples 1 à 8 et aux exemples comparatifs 2, 3, 4 et 5.

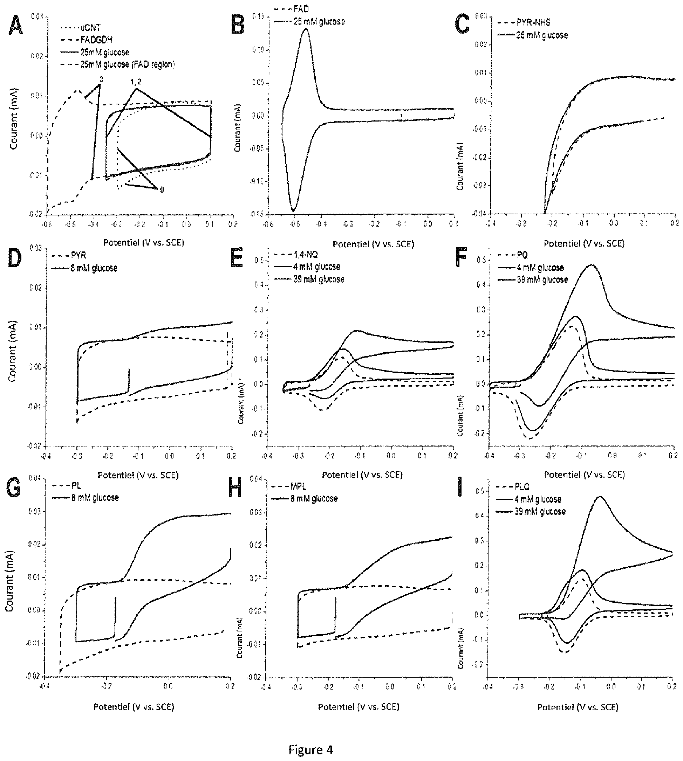

La figure 4 montre les voltampérogrammes cycliques obtenus avec chacune des électrodes obtenues aux exemples 1, 4 à 8 et aux exemples comparatifs 2, 4 à 6 ainsi que l'enzyme FAD-GDH seule.

Dans la figure 4, les termes suivants ont les significations suivantes :
- uCNT = nanotubes de carbone seuls,
- FADGDH = CNTs fonctionnalisés par la FAD-GDH,
- 25 mM glucose = CNTs fonctionnalisés par la FAD-GDH en présence d'une solution de glucose à 25 mmol L⁻¹,
- 25 mM glucose (FAD region) = idem que précédemment en incluant la région électroactive du cofacteur de la FAD-GDH (la FAD) dans la fenêtre de mesure en présence d'une solution de glucose à 25 mmol L⁻¹,
- FAD = Flavine Adénine Dinucléotide,
- 25 mM glucose = concentration de la solution en glucose de 25 mmol L⁻¹,
- PYR-NHS = ester N-hydroxysuccinimide de l'acide pyrène butyrique,
- PYR = pyrène,
- 1,4-NQ = 1,4-naphthoquinone,
- PQ = 9,10-phénanthrènequinone,
- PL= 1,10-phénanthroline,
- MPL = 5-méthyl-1,10-phénanthroline,
- PLQ = 1,10-phénanthroline-5,6-dione.

La cartouche notée A dans la figure 4 montre les voltampérogrammes cycliques obtenus pour des électrodes obtenues aux exemples comparatifs 2 et 4.

L'électrode testée était une électrode de carbone vitreux (support 1) recouverte de la couche 2 de nanotubes de carbone seule (exemple comparatif 2) ou dans laquelle l'enzyme FAD-GDH seule a été déposée sur la couche 2 de nanotubes de carbone (exemple comparatif 4).

On constate à partir du voltampérogramme montré en cartouche A qu'il n'y a aucun signal redox visible (entre -0,300 et 0,100 V vs ECS ; ECS : électrode de référence au calomel saturé [équivalent français de SCE pour « saturated calomel électrode » en anglais]) sur une électrode de carbone vitreux recouverte d'une couche 2 de nanotubes de carbone (courbe 0 en pointillés).

On constate également à partir du voltampérogramme montré en cartouche A qu'il n'y a aucune variation du courant catalytique en l'absence ou en présence de glucose pour une électrode de carbone vitreux recouverte d'une couche 2 de nanotubes de carbone modifiée directement par la couche 4 d'enzyme FAD-GDH (la courbe 1 en traits pointillés a été obtenue en l'absence de glucose et la courbe en trait plein notée 2 est la courbe obtenue en présence de glucose ; 1 et 2 se superposent parfaitement).

La courbe en tirets notée 3 représente le voltampérogramme cyclique obtenu avec une fenêtre électrochimique plus large (de -0,600 à 0,100 V vs ECS), en présence d'une solution de glucose à 25 mmol L⁻¹.

On observe sur la courbe 3 la présence d'un pic redox centré sur -0,481 V vs. ECS.

Ce pic est attribué à la réponse électrochimique du centre actif de l'enzyme FAD-GDH, la FAD, pour des enzymes partiellement ou complètement dénaturées, ce qui montre que, grâce à sa structure, le cofacteur de l'enzyme peut aussi être adsorbé à la surface des nanotubes de carbone. En revanche, cette FAD seule ne peut en aucun cas être le siège d'une réaction électrocatalytique puisque la structure intacte de l'enzyme est un élément clé de la catalyse enzymatique.

Ceci est confirmé par le voltampérogramme montré dans la cartouche B de la figure 4.

Ce voltampérogramme a été obtenu pour l'électrode obtenue en exemple comparatif 5 et composée des couches 2, 3 et 4, respectivement, de nanotubes de carbone, de molécules aromatiques et d'enzymes FAD (sans ou en présence de glucose à 25 mmol L⁻¹ ; courbes 1 et 2 identiques), avec la fenêtre électrochimique de -0,600 à 0,100 V vs ECS identique à celle utilisée par la courbe 3 de la cartouche A.

Le fait de trouver un potentiel redox identique pour les voltampérogrammes des cartouches A et B renseignent sur l'intégrité des enzymes sur l'électrode.

Il est largement référencé dans la littérature que l'enzyme FAD, pour une enzyme fonctionnelle, a un potentiel plus élevé que la FAD libre en solution.

Pour cette raison, il est raisonnable de conclure que le pic présent en cartouche A n'est en fait du qu'à la FAD libre issue de la FAD-GDH dont la structure aurait été altérée et donc des enzymes inactives.

La FAD seule ne peut pas suffire pour oxyder le glucose. Pour cela, il est impératif que la FAD soit à l'intérieur de la structure protéique.

En figure 4, la cartouche C représente les voltampérogrammes cycliques de l'électrode de l'exemple comparatif 6 constituée d'une électrode de carbone vitreux, de la couche 2 de nanotubes de carbone fonctionnalisés par la couche 3 d'une molécule aromatique qui est l'ester N-hydroxysuccinimide de l'acide pyrène butyrique.

Cette molécule aromatique ne fait pas partie des molécules utilisables dans l'invention bien que la fonction ester N-hydrosuccinimide soit régulièrement utilisée pour réaliser des couplages de type peptidique entre un acide carboxylique et une fonction amine. On devrait donc avoir la fonction N-hydroxysuccinimide qui réagit avec les fonctions -NH₂ situées sur la périphérie de l'enzyme FAD-GDH et ainsi immobiliser de manière covalente et avec une orientation totalement aléatoire cette enzyme sur l'électrode. Comme le montre le voltampérogramme de la cartouche C, la restriction de mouvement lors de la formation de la liaison covalente qui aurait pu permettre la reconnaissance « clé-serrure » n'est plus réalisable.

Les cartouches D, G et H représentent les voltampérogrammes cycliques des électrodes obtenues aux exemples 4, 5 et 6 respectivement.

Toutes les molécules aromatiques utilisées dans ces exemples sont des molécules aromatiques non-électroactives.

En effet, aucun signal redox n'est observé pour de telles électrodes en absence de glucose (on retrouve le même signal que pour les nanotubes de carbone non fonctionnalisés) tandis qu'un signal électrocatalytique est repéré lors de l'ajout de glucose (solution à 8 mmol L⁻¹). Ce phénomène peut donc complètement s'apparenter à du transfert électronique direct (DET) grâce à l'interaction « clé-serrure » jouée entre la molécule aromatique et le centre hydrophobe de l'enzyme et qui corrobore le fait que la 1,10-phénanthroline est un inhibiteur de la FAD-GDH.

La molécule aromatique étant immobilisée sur les tubes par interactions π- π, elle va avoir tendance à attirer à elle le centre actif de l'enzyme, sans pour autant provoquer son inhibition.

Les cartouches E, F et I montrent les voltampérogrammes cycliques obtenus avec des électrodes obtenues aux exemples 7, 8 et 1 respectivement.

On voit à partir de ces voltampérogrammes cycliques que toutes les quinones montrent un comportement de médiateurs redox pour le transfert électronique médié (MET) de l'enzyme FAD-GDH.

Les courants électrocatalytiques sont nettement supérieurs à ceux obtenus par transfert électronique direct (DET).

La forme des voltampérogrammes cycliques, notamment pour l'électrode ayant pour molécule aromatique la 1,10-phénanthroline-5,6-dione de l'exemple 1, indique qu'à faible concentration de glucose (<4 mmol L⁻¹) une partie du courant électrocatalytique pourrait également provenir d'un transfert électronique direct.

La 1,10-phénanthroline-5,6-dione apparaît donc être la meilleure molécule aromatique redox pour l'oxydation électrocatalytique de l'enzyme FAD-GDH en utilisant une immobilisation non-covalente sur une couche de nanotubes de carbone.

En effet, les courants électrocatalytiques mesurés pour les électrodes avec comme molécules aromatiques la 1,4-naphtaquinone molécules aromatiques (exemple 7) et la 9,10-phénanthroquinone (exemple 8) ne dépassent pas respectivement 200 µA, et 300 µA pour des potentiels supérieurs à 0 V vs ECS, cette valeur de 300 µA étant cependant tout à fait exploitable, après saturation (39 mmol L⁻¹ de glucose).

La 1,10-phénanthroline-5,6-dione présente donc de meilleures performances avec une saturation proche de 130 mmol L⁻¹ de glucose et des courants proches de 600-700 µA.

**Exemple 13** : Caractérisation électrochimique des électrodes obtenues à l'exemple comparatif 4.

La figure 5 montre les voltampérogrammes cycliques obtenus avec chacune des électrodes obtenues suivant l'exemple comparatif 4.

Dans la figure 5, les termes suivants ont les significations suivantes :
0µM PX = CNTs fonctionnalisés par la FAD-GDH en présence d'une solution de glucose à 100 mmol L⁻¹ et en absence de quelconque molécules aromatiques (X représentant la molécule aromatique PLQ (cartouche A) ou PQ (cartouche B))

400µM PX = CNTs fonctionnalisés par la FAD-GDH en présence d'une solution de glucose à 100 mmol L⁻¹ et de 400 µmol L⁻¹ d'une molécule aromatique PLQ (cartouche A) ou PQ (cartouche B)

400µM PX (180min) = CNTs fonctionnalisés par la FAD-GDH en présence d'une solution de glucose à 100 mmol L⁻¹ et de 200 µmol L⁻¹ d'une molécule aromatique PLQ (cartouche A) ou PQ (cartouche B) après une incubation dans la solution d'une durée de 180 minutes.

200µM PQ = CNTs fonctionnalisés par la FAD-GDH en présence d'une solution de glucose à 100 mmol L⁻¹ et de 200 µmol L⁻¹ d'une molécule aromatique PQ (cartouche C et D)

200µM PQ + 200µM PY (180min) = CNTs fonctionnalisés par la FAD-GDH en présence d'une solution de glucose à 100 mmol L⁻¹, de 200 µmol L⁻¹ d'une molécule aromatique PQ et de 200 µmol L⁻¹ d'une molécule aromatique PY après une incubation dans la solution d'une durée de 180 minutes. (Y représentant la molécule aromatique PL (cartouche C) ou PH (cartouche D)).
- PLQ = 1,10-phénanthroline-5,6-dione.
- PQ = 9,10-phénanthrènequinone,
- PL = 1,10-phénanthroline,
- PH = phénanthrène,

La cartouche notée A dans la figure 5 montre les voltampérogrammes cycliques obtenus pour des électrodes obtenues à l'exemple comparatif 4.

L'électrode testée était une électrode de carbone vitreux (support 1) dans laquelle l'enzyme FAD-GDH seule a été déposée sur la couche 2 de nanotubes de carbone (exemple comparatif 4).

On constate à partir du voltampérogramme montré en cartouche A une forte augmentation du courant catalytique entre -0,250 et 0,150 V vs ECS sur une électrode de carbone vitreux dans laquelle l'enzyme FAD-GDH seule a été déposée sur la couche 2 de nanotubes de carbone dès lors que PLQ est en présence (trait plein noir) opposé à l'absence de médiateur rédox en solution (trait pointillé noir).

On constate également à partir du voltampérogramme montré en cartouche A qu'il y a une forte diminution du courant catalytique après 180 minutes d'immersion de l'électrode dans la solution contenant 100 mmol L¹ de glucose et 400 µmol L⁻¹ de PLQ pour la même électrode de carbone vitreux recouverte d'une couche 2 de nanotubes de carbone modifiée directement par la couche 4 d'enzyme FAD-GDH. Ceci suggère une progressive inhibition de l'enzyme par la PLQ (analogue électroactif de PL; inhibiteur cité par la compagnie fournissant l'enzyme).

De la même façon, on remarque en cartouche B, C et D une forte augmentation du courant catalytique entre -0,300 et 0,150 V vs ECS sur une électrode de carbone vitreux dans laquelle l'enzyme FAD-GDH seule a été déposée sur la couche 2 de nanotubes de carbone dès lors que PQ est en présence (trait plein noir) opposé à l'absence de médiateur rédox en solution (trait pointillé noir).

En revanche, dans ce cas présent, aucune diminution du courant catalytique n'est observé après une incubation de 180 min de l'électrode décrite précédemment dans une solution contenant 100 mmol L¹ de glucose et 400 µmol L⁻¹ de PQ.

Ce constat reste valide lorsque la solution se compose de 100 mmol L¹ de glucose et 200 µmol L⁻¹ de PQ et de 200 µmol L⁻¹ de PH (analogue de non électroactif de PQ).

Le phénomène est inversé lorsque la solution se compose de 100 mmol L⁻¹ de glucose et 200 µmol L⁻¹ de PQ et de 200 µmol L⁻¹ de PL. Le courant catalytique chute de nouveau et le phénomène d'inhibition décrite par le fabricant de la FAD-GDH est obtenu.

## Revendications

1. Bioélectrode **caractérisée en ce qu'**elle comprend la superposition de :
- une couche (2) de nanotubes de carbone,
- une couche (3), déposée sur la couche (2) de nanotubes de carbone, constituée de molécules aromatiques choisies dans le groupe formé par la 9,10-phénanthrènequinone, la 1,10-phénanthroline-5,6-dione, la 9,10-anthraquinone, la phénanthrène, la 1,10-phénanthroline, la 5-méthyl-1,10-phénanthroline, le pyrène, le 1-aminopyrène, l'acide pyrène-1-butyrique, et les mélanges de deux ou plus de ceux-ci,
- une couche (4) d'enzymes Flavine Adénine Dinucléotide-Glucose Déhydrogènase (FAD-GDH) adsorbée sur la couche (3) des molécules aromatiques .

2. Bioélectrode selon la revendication 1, **caractérisée en ce qu'**elle comprend de plus une couche (6) en un matériau poreux et optionnellement hydrophile recouvrant au moins la surface libre de la couche (4) d'enzymes FAD-GDH, de préférence la couche (6) est en alginate, chitosan, Nafion® ou gel de silice.

3. Bioélectrode selon la revendication 1, **caractérisée en ce que** la couche (4) est constituée de molécules de 9,10-phénanthrènequinone ou de 1,10-phénanthroline-5,6-dione.

4. Bioélectrode selon l'une quelconque des revendications précédentes, **caractérisée en ce que** la couche (2) de nanotubes de carbone comprend entre 2,5 µg et 510 µg de nanotubes de carbone par cm² de surface de couche, de préférence entre 2,5 et 100 µg de nanotubes de carbone par cm² de surface de la couche (2).

5. Bioélectrode selon l'une quelconque des revendications précédentes, **caractérisée en ce qu'**elle comprend de plus un support (1) en un matériau conducteur d'électrons, de préférence en carbone vitreux, sous la couche (2) de nanotubes de carbone.

6. Bioélectrode selon l'une quelconque des revendications 1 à 4, **caractérisée en ce qu'**elle comprend de plus un fil en un matériau électriquement conducteur relié à la surface de la couche (2) de nanotubes de carbone qui n'est pas recouverte de la couche (3) de molécules aromatiques .

7. Bioélectrode selon l'une quelconque des revendications précédentes, **caractérisée en ce qu'**elle comprend de plus une couche (5) d'enrobage imperméable aux fluides, de préférence en un matériau siliconé, entourant la superposition des couches (2), (3) et (4) et optionnellement du support 1.

8. Dispositif comprenant une bioélectrode selon l'une quelconque des revendications 1 à 7.

9. Dispositif selon la revendication 8, **caractérisé en ce qu'**il est une cellule enzymatique à biocarburant.

10. Dispositif comprenant une bioélectrode selon l'une quelconque des revendications 1 à 6, **caractérisé en ce qu'**il est un dispositif de détection du glucose.

11. Utilisation d'une bioélectrode selon l'une quelconque des revendications 1 à 6 ou d'un dispositif selon la revendication 8 pour l'oxydation du glucose.

12. Procédé de fabrication d'une bioélectrode selon l'une quelconque des revendications 1 à 7, **caractérisé en ce qu'**il comprend les étapes suivantes :
a) préparation d'une suspension de nanotubes de carbone ayant une concentration en nanotubes de carbone comprise entre 0,5 mg.mL⁻¹ et 10 mg.mL⁻¹, et de préférence de 2,5 mg.mL⁻¹, dans un solvant organique choisi parmi la N-méthyl-2-pyrrolidone (NMP), le dichlorométhane (DCM), l'acétonitrile (ACN), le 1,3-dioxolane (DXL), le diméthylformamide (DMF), et les mélanges de deux ou plus de ceux-ci, et de préférence la NMP,
b) dépôt d'une goutte (drop-casting) de la suspension de nanotubes de carbone obtenue à l'étape a) sur un support (1) en un matériau conducteur d'électrons, de préférence en carbone vitreux,
c) évaporation du solvant organique de la suspension de nanotubes de carbone, ce par quoi une couche (2) de nanotubes de carbone est formée sur le support (1),
d) préparation d'une solution comprenant des molécules aromatiques, choisies dans le groupe consistant en la 9,10-phénanthrenequinone, la 1,10-phénanthroline-5,6-dione, la 9,10-anthraquinone, la phénanthrène, la 1,10-phénanthroline, la 5-méthyl-1,10-phénanthroline, le pyrène, le 1-aminopyrène, l'acide pyrène-1-butyrique, et les mélanges de deux ou plus de celles-ci et ayant une concentration comprise entre 0,5 mmol.L⁻¹ et 15 mmol.L⁻¹, et de préférence 2,5 mmol.L⁻¹ de molécules aromatiques dissoutes dans un solvant organique identique ou différent du solvant organique de la suspension de nanotubes de carbone, le solvant étant choisi parmi la NMP, le DCM, l'ACN, le DXL, le DMF et les mélanges de deux ou plus de ceux-ci, et étant de préférence de la NMP,
e) dépôt sur une surface de la couche (2) de nanotubes de carbone obtenue à l'étape c) de la solution de molécules aromatiques obtenues à l'étape d), par dépôt d'une goutte, ayant de préférence un volume compris entre 5 et 200 µL, plus préférablement un volume de 175 µL, dela solution de molécules aromatiques sur la surface de la couche (2), ou par incubation d'au moins la surface libre de la couche (2) dans, la solution de molécules aromatiques obtenue à l'étape d),
f) évaporation du solvant de la solution de molécules aromatiques déposée à l'étape e), ce par quoi une couche (3) de molécules aromatiquesest formée sur la couche (2),
g) préparation d'une solution aqueuse d'enzymes FAD-GDH ayant une concentration en enzymes FAD-GDH comprise entre 0,1 mg.mL⁻¹ et 5 mg.mL⁻¹, plus préférablement de 0,75 mg.mL⁻¹,
h) dépôt sur la couche (3) de molécules aromatiques obtenue à l'étape f), de la solution d'enzymes FAD-GDH obtenue à l'étape g), par incubation d'au moins la surface libre de la couche (3) de molécules aromatiques dans une solution aqueuse contenant les enzymes FAD-GDH obtenue à l'étape g), ou par dépôt d'une goutte (drop-casting) de ladite solution aqueuse contenant les enzymes FAD-GDH obtenue à l'étape f) sur la surface libre de la couche (3).

13. Procédé de fabrication d'une bioélectrode selon la revendication 12, **caractérisé en ce que** l'étape a) de dépôt d'une goutte de la dispersion de nanotubes de carbone est répétée au moins une fois.

14. Procédé de fabrication d'une bioélectrode selon l'une quelconque des revendications1 à7 **caractérisé en ce qu'**il comprend les étapes suivantes :
A) préparation d'une suspension de nanotubes de carbone dans un solvant organique de préférence choisi parmi la NMP, le DCM, l'ACN, le DXL, le DMF et les mélanges de deux ou plus de ceux-ci, plus préférablement du DMF, la suspension ayant une concentration en nanotubes de carbone comprise entre 1 mg.mL⁻¹ et 15 mg.mL⁻¹, de préférence de 1 mg.mL⁻¹,
B) préparation d'une solution de molécules aromatiques choisies dans le groupe constitué par la 9,10-phénanthrènequinone, la 1,10-phénanthroline-5,6-dione, la 9,10-anthraquinone, la phénanthrène, la 1,10-phénanthroline, la 5-méthyl-1,10-phénanthroline, le pyrène, le 1-aminopyrène, l'acide pyrène-1-butyrique, et les mélanges de deux ou plus de celles-ci, de préférence de 1,10-phénanthroline-5,6-dione, dans un solvant organique choisi parmi la NMP, le DCM, l'ACN, le DXL, le DMF et les mélanges de deux ou plus de ceux-ci et identique ou différent du solvant organique de la suspension de nanotubes de carbone obtenue à l'étape a), de préférence du DMF, cette solution de molécules aromatiques ayant une concentration en molécules aromatiques comprise entre 0,5 mmol.L⁻¹ et 15 mmol.L⁻¹, de préférence 2,5 mmol.L⁻¹,
C) ajout de la solution de molécules aromatiques obtenue à l'étape b) dans la suspension de nanotubes obtenue à l'étape A), et mélange,
D) filtration de la dispersion obtenue à l'étape C) à travers un support filtrant,
E) évaporation du solvant de la suspension de nanotubes de carbone et du solvant de la solution de molécules médiatrices aromatiques, ce par quoi une couche (2) de nanotubes de carbone, elle-même recouverte sur au moins une de ses surfaces d'une couche (3) desdites molécules aromatiques est formée,
F) préparation d'une solution aqueuse d'enzymes FAD-GDH ayant une concentration en enzymes FAD-GDH comprise entre 0,1 et 5 mg.mL⁻¹,
G) dépôt d'une goutte de 150 µL de la solution d'enzymes FAD-GDH préparée à l'étape F) sur la surface libre de la couche (3) formant ainsi une couche (4) d'enzymes FAD-GDH adsorbés sur la couche (3) de molécules aromatiques,
H) retrait de l'assemblage obtenu à l'étape G du support filtrant.

15. Procédé de fabrication d'une bioélectrode selon l'une quelconque des revendications 1 à 7, **caractérisé en ce qu'**il comprend les étapes suivantes :
al) préparation d'une suspension de nanotubes de carbone dans un solvant organique, de préférence du DMF, ayant une concentration en nanotubes de carbone comprise entre 1 mg.mL⁻¹ et 15 mg.mL⁻¹, de préférence de 1 mg.mL⁻¹,
b1) préparation d'une solution de molécules aromatiques choisies dans le groupe constitué par la 9,10-phénanthrènequinone, la 1,10-phénanthroline-5,6 dione, 9,10-anthraquinone, la phénanthrène, la 1,10-phénanthroline, la 5-méthyl-1,10-phénanthroline, le pyrène, le 1-aminopyrène, l'acide pyrène-1-butyrique, et les mélanges de deux ou plus de celles-ci, de préférence de 1,10-phénanthroline-5,6 dione, dans un solvant organique identique ou différent du solvant organique de la suspension de nanotubes de carbone obtenue à l'étape a1), de préférence du DMF, cette solution ayant une concentration en molécules aromatiques comprise entre 0,5 et 15 mmol.L⁻¹, de préférence de 2,5 mmol.L⁻¹,
c1) filtration de la dispersion obtenue à l'étape b1) à travers un support (1) filtrant,
d1) évaporation du solvant de la dispersion de nanotubes de carbone déposée à l'étape c) ce par quoi une couche (2) de nanotubes de carbone est formée,
e1) retrait de la couche de nanotubes de carbone obtenue à l'étape d1) du support (1) filtrant,
f1) dépôt d'une goutte de 150 µL de la solution de molécules aromatiques obtenue à l'étape b1) sur au moins une surface de la couche (2) de nanotubes de carbone obtenue à l'étape e1),
g1) évaporation du solvant de la solution de molécules aromatiques déposée à l'étape f1), ce par quoi une couche (3) desdites molécules aromatiques est formée sur la couche (2) de nanotubes de carbone,
h1) préparation d'une solution aqueuse d'enzymes FAD-GDH, ayant une concentration en enzymes FAD-GDH comprise entre 0,1 et 5 mg.mL⁻¹,
il) dépôt d'une goutte de 150 µL de la solution d'enzymes FAD-GDH obtenue à l'étape h) sur au moins une surface de la couche (3) de molécules aromatiques, formant ainsi une couche (4) d'enzymes FAD-GDH adsorbés sur la couche (3).

16. Procédé de fabrication d'une bioélectrode selon la revendication 14 ou 15, **caractérisé en ce qu'**il comprend de plus, une étape k1) de fixation d'un fil en un matériau électriquement conducteur sur la surface de la couche (2) de nanotubes de carbone non recouverte de la couche (3) de molécules aromatiques, cette étape étant mise en œuvre avant ou après l'étape G) ou i1) de dépôt de la couche (4) d'enzymes FAD-GDH.

17. Procédé de fabrication d'une bioélectrode selon l'une quelconque des revendications 12 à 16, **caractérisé en ce qu'**il comprend de plus une étape de dépôt d'une couche (6) en un matériau poreux et optionnellement hydrophile sur au moins la surface libre de la couche (4).

18. Procédé de fabrication d'une bioélectrode selon l'une quelconque des revendications 12 à 17, **caractérisé en ce qu'**il comprend de plus une étape d'enrobage de la superposition : couche (2) de nanotubes de carbone/couche (3) de molécules aromatiques / couche (4) d'enzymes FAD-GDH / optionnellement support (1) / fil en un matériau électriquement conducteur, avec un matériau imperméable aux fluides, de préférence à base de silicone.

## Patentansprüche

1. Bioelektrode, **dadurch gekennzeichnet, dass** sie übereinanderliegend umfasst:
- eine Schicht (2) aus Kohlenstoff-Nanoröhren,
- eine auf der Schicht (2) aus Kohlenstoff-Nanoröhren abgeschiedene Schicht (3), bestehend aus aromatischen Molekülen, ausgewählt aus der Gruppe, bestehend aus 9,10-Phenanthrenchinon, 1,10-Phenanthrolin-5,6-dion, 9,10-Anthrachinon, Phenanthren, 1,10-Phenanthrolin, 5-Methyl-1,10-phenanthrolin, Pyren, 1-Aminopyren, Pyren-1-buttersäure und Gemischen aus zwei oder mehreren davon,
- eine Schicht (4) aus Flavin-Adenin-Dinucleotid-Glucose-Dehydrogenase (FAD-GDH)-Enzymen, die auf der Schicht (3) aus aromatischen Molekülen adsorbiert ist.

2. Bioelektrode nach Anspruch 1, **dadurch gekennzeichnet, dass** sie weiterhin eine Schicht (6) aus einem porösen und gegebenenfalls hydrophilen Material umfasst, die mindestens die freie Oberfläche der Schicht (4) aus FAD-GDH-Enzymen bedeckt, wobei die Schicht (6) vorzugsweise aus Alginat, Chitosan, Nafion® oder Kieselgel besteht.

3. Bioelektrode nach Anspruch 1, **dadurch gekennzeichnet, dass** die Schicht (4) aus Molekülen von 9,10-Phenanthrenchinon oder 1,10-Phenanthrolin-5,6-dion besteht.

4. Bioelektrode nach einem der vorangehenden Ansprüche, **dadurch gekennzeichnet, dass** die Schicht (2) aus Kohlenstoff-Nanoröhren zwischen 2,5 µg und 510 µg Kohlenstoff-Nanoröhren pro cm² Oberfläche der Schicht, vorzugsweise zwischen 2,5 und 100 µg Kohlenstoff-Nanoröhren pro cm² Oberfläche der Schicht (2) umfasst.

5. Bioelektrode nach einem der vorangehenden Ansprüche, **dadurch gekennzeichnet, dass** sie weiterhin einen Träger (1) aus einem elektronenleitenden Material, vorzugsweise aus Glaskohlenstoff, unter der Schicht (2) aus Kohlenstoff-Nanoröhren umfasst.

6. Bioelektrode nach einem der Ansprüche 1 bis 4, **dadurch gekennzeichnet, dass** sie weiterhin einen Draht aus einem elektrisch leitfähigen Material umfasst, der mit der Oberfläche der Schicht (2) aus Kohlenstoff-Nanoröhren verbunden ist, die nicht mit der Schicht (3) aus aromatischen Molekülen bedeckt ist.

7. Bioelektrode nach einem der vorangehenden Ansprüche, **dadurch gekennzeichnet, dass** sie weiterhin eine flüssigkeitsundurchlässige Überzugsschicht (5), vorzugsweise aus einem Silikonmaterial, umfasst, die die übereinanderliegenden Schichten (2), (3) und (4) und gegebenenfalls den Träger 1 umgibt.

8. Vorrichtung, umfassend eine Bioelektrode nach einem der Ansprüche 1 bis 7.

9. Vorrichtung nach Anspruch 8, **dadurch gekennzeichnet, dass** sie eine Biokraftstoff-Enzymzelle ist.

10. Vorrichtung, umfassend eine Bioelektrode nach einem der Ansprüche 1 bis 6, **dadurch gekennzeichnet, dass** sie eine Vorrichtung zum Nachweis von Glucose ist.

11. Verwendung einer Bioelektrode nach einem der Ansprüche 1 bis 6 oder einer Vorrichtung nach Anspruch 8 für die Oxidation von Glucose.

12. Verfahren zur Herstellung einer Bioelektrode nach einem der Ansprüche 1 bis 7, **dadurch gekennzeichnet, dass** es die folgenden Schritte umfasst:
a) Herstellung einer Suspension von Kohlenstoff-Nanoröhren, die eine Kohlenstoff-Nanoröhren-Konzentration zwischen 0,5 mg.ml⁻¹ und 10 mg.ml⁻¹ und vorzugsweise 2,5 mg.ml⁻¹ umfasst, in einem organischen Lösungsmittel, ausgewählt aus N-Methyl-2-pyrrolidon (NMP), Dichlormethan (DCM), Acetonitril (ACN), 1,3-Dioxolan (DXL), Dimethylformamid (DMF) und Gemischen aus zwei oder mehreren davon, und vorzugsweise NMP,
b) Abscheidung eines Tropfens (Drop-casting) der in Schritt a) erhaltenen Suspension von Kohlenstoff-Nanoröhren auf einem Träger (1) aus einem elektronenleitenden Material, vorzugsweise Glaskohlenstoff,
c) Verdampfung des organischen Lösungsmittels aus der Suspension von Kohlenstoff-Nanoröhren, wodurch eine Schicht (2) aus Kohlenstoff-Nanoröhren auf dem Träger (1) gebildet wird,
d) Herstellung einer Lösung, die aromatische Moleküle umfasst, ausgewählt aus der Gruppe, bestehend aus 9,10-Phenanthrenchinon, 1,10-Phenanthrolin-5,6-dion, 9,10-Anthrachinon, Phenanthren, 1,10-Phenanthrolin, 5-Methyl-1,10-phenanthrolin, Pyren, 1-Aminopyren, Pyren-1-buttersäure und Gemischen aus zwei oder mehreren davon, und die eine Konzentration zwischen 0,5 mMol.l⁻¹ und 15 mMol.l⁻¹ und vorzugsweise 2,5 mMol.l⁻¹ aromatischer Moleküle umfasst, die in dem gleichen oder verschiedenen organischen Lösungsmittel wie das organische Lösungsmittel der Suspension von Kohlenstoff-Nanoröhren gelöst sind, wobei das Lösungsmittel aus NMP, DCM, ACN, DXL, DMF und Gemischen aus zwei oder mehreren davon ausgewählt ist und vorzugsweise NMP ist,
e) Abscheidung der in Schritt c) erhaltenen Lösung aromatischer Moleküle auf einer Oberfläche der Schicht (2) aus Kohlenstoff-Nanoröhren, die in Schritt d) erhalten wurde, durch Abscheidung eines Tropfens der Lösung aromatischer Moleküle mit einem Volumen, das vorzugsweise zwischen 5 und 200 µl, bevorzugter einem Volumen von 175 µl, umfasst, auf der Oberfläche der Schicht (2), oder durch Inkubation mindestens der freien Oberfläche der Schicht (2) in der in Schritt d) erhaltenen Lösung aromatischer Moleküle,
f) Verdampfung des Lösungsmittels aus der in Schritt e) abgeschiedenen Lösung aromatischer Moleküle, wobei auf der Schicht (2) eine Schicht (3) aromatischer Moleküle gebildet wird,
g) Herstellung einer wässrigen Lösung von FAD-GDH-Enzymen, die eine Konzentration von FAD-GDH-Enzymen zwischen 0,1 mg.ml⁻¹ und 5 mg.ml⁻¹, bevorzugter 0,75 mg.ml⁻¹, umfasst,
h) Abscheidung auf der in Schritt f) erhaltenen Schicht (3) aromatischer Moleküle der in Schritt g) erhaltenen Lösung der FAD-GDH-Enzyme, durch Inkubation mindestens der freien Oberfläche der Schicht (3) aromatischer Moleküle in einer wässrigen Lösung, die die in Schritt g) erhaltenen FAD-GDH-Enzyme enthält, oder durch Abscheidung eines Tropfens (Drop-casting) dieser wässrigen Lösung, die die in Schritt f) erhaltenen FAD-GDH-Enzyme enthält, auf der freien Oberfläche der Schicht (3).

13. Verfahren zur Herstellung einer Bioelektrode nach Anspruch 12, **dadurch gekennzeichnet, dass** der Schritt a) des Abscheidens eines Tropfens der Dispersion von Kohlenstoff-Nanoröhren mindestens einmal wiederholt wird.

14. Verfahren zur Herstellung einer Bioelektrode nach einem der Ansprüche 1 bis 7, **dadurch gekennzeichnet, dass** es die folgenden Schritte umfasst:
A) Herstellung einer Suspension von Kohlenstoff-Nanoröhren in einem organischen Lösungsmittel, vorzugsweise ausgewählt aus NMP, DCM, ACN, DXL, DMF und Gemischen von zwei oder mehreren davon, bevorzugter DMF, wobei die Suspension eine Kohlenstoff-Nanoröhren-Konzentration zwischen 1 mg.ml⁻¹ und 15 mg.ml⁻¹, vorzugsweise 1 mg.ml⁻¹, umfasst,
B) Herstellung einer Lösung von aromatischen Molekülen, ausgewählt aus der Gruppe, bestehend aus 9,10-Phenanthrenchinon, 1,10-Phenanthrolin-5,6-dion, 9,10-Anthrachinon, Phenanthren, 1,10-Phenanthrolin, 5-Methyl-1,10-phenanthrolin, Pyren, 1-Aminopyren, Pyren-1-buttersäure und Gemischen von zwei oder mehreren davon, vorzugsweise 1,10-Phenanthrolin-5,6-dion, in einem organischen Lösungsmittel, ausgewählt aus NMP, DCM, ACN, DXL, DMF und Gemischen aus zwei oder mehreren davon und gleich oder verschieden von dem organischen Lösungsmittel der in Schritt a) erhaltenen Suspension von Kohlenstoff-Nanoröhren, vorzugsweise DMF, wobei die Lösung aromatischer Moleküle eine Konzentration aromatischer Moleküle zwischen 0,5 mMol.l⁻¹ und 15 mMol.l⁻¹, vorzugsweise 2,5 mMol.l⁻¹, umfasst,
C) Hinzufügung der in Schritt b) erhaltenen Lösung aromatischer Moleküle zu der in Schritt A) erhaltenen Suspension von Nanoröhren und Mischen,
D) Filtration der in Schritt C) erhaltenen Dispersion durch einen Filterträger,
E) Verdampfung des Lösungsmittels der Suspension von Kohlenstoff-Nanoröhren und des Lösungsmittels der Lösung von aromatischen Vermittlermolekülen, wodurch eine Schicht (2) von Kohlenstoff-Nanoröhren gebildet wird, die ihrerseits auf mindestens einer ihrer Oberflächen mit einer Schicht (3) der gebildeten aromatischen Moleküle bedeckt ist,
F) Herstellung einer wässrigen Lösung von FAD-GDH-Enzymen mit einer Konzentration von FAD-GDH-Enzymen, die zwischen 0,1 und 5 mg.ml⁻¹ umfasst,
G) Abscheidung eines 150 µl Tropfens der in Schritt F) hergestellten FAD-GDH-Enzymlösung auf der freien Oberfläche der Schicht (3), wodurch eine Schicht (4) aus FAD-GDH-Enzymen gebildet wird, die an der Schicht (3) aus aromatischen Molekülen adsorbiert wird,
H) Entfernung der in Schritt G erhaltenen Baugruppe vom Filterträger.

15. Verfahren zur Herstellung einer Bioelektrode nach einem der Ansprüche 1 bis 7, **dadurch gekennzeichnet, dass** es die folgenden Schritte umfasst:
a1) Herstellung einer Suspension von Kohlenstoff-Nanoröhren in einem organischen Lösungsmittel, vorzugsweise DMF, die eine Kohlenstoff-Nanoröhren-Konzentration zwischen 1 mg.ml⁻¹ und 15 mg.ml⁻¹, vorzugsweise 1 mg.ml⁻¹, umfasst
b1) Herstellung einer Lösung von aromatischen Molekülen, ausgewählt aus der Gruppe, bestehend aus 9,10-Phenanthrenchinon, 1,10-Phenanthrolin-5,6-dion, 9,10-Anthrachinon, Phenanthren, 1,10-Phenanthrolin, 5-Methyl-1,10-phenanthrolin, Pyren, 1-Aminopyren, Pyren-1-buttersäure und Gemischen von zwei oder mehreren davon, vorzugsweise 1,10-Phenanthrolin-5,6-dion, in einem organischen Lösungsmittel, das gleich oder verschieden von dem organischen Lösungsmittel der in Schritt a1) erhaltenen Suspension von Kohlenstoff-Nanoröhren ist, vorzugsweise DMF, wobei diese Lösung eine Konzentration an aromatischen Molekülen zwischen 0,5 und 15 mMol.l⁻¹, vorzugsweise 2,5 mMol.l⁻¹, umfasst,
c1) Filtration der in Schritt b1) erhaltenen Dispersion durch einen Filterträger (1), d1) Verdampfung des Lösungsmittels aus der in Schritt c) abgeschiedenen Dispersion von Kohlenstoff-Nanoröhren, wodurch eine Schicht (2) aus Kohlenstoff-Nanoröhren gebildet wird,
e1) Entfernung der in Schritt d1) erhaltenen Schicht von Kohlenstoff-Nanoröhren vom Filterträger (1),
f1) Abscheidung eines 150 µl Tropfens der in Schritt b1) erhaltenen Lösung aromatischer Moleküle auf mindestens einer Oberfläche der in Schritt e1) erhaltenen Schicht (2) von Kohlenstoff-Nanoröhren,
g1) Verdampfung des Lösungsmittels aus der in Schritt f1) abgeschiedenen Lösung aromatischer Moleküle, wobei eine Schicht (3) der aromatischen Moleküle auf der Schicht (2) aus Kohlenstoff-Nanoröhren gebildet wird,
h1) Herstellung einer wässrigen Lösung von FAD-GDH-Enzymen, die eine FAD-GDH-Enzymkonzentration zwischen 0,1 und 5 mg.ml⁻¹ umfasst,
i1) Abscheidung eines 150 µl Tropfens der in Schritt h) erhaltenen FAD-GDH-Enzymlösung auf mindestens eine Oberfläche der Schicht (3) aus aromatischen Molekülen, wodurch eine Schicht (4) aus FAD-GDH-Enzymen gebildet wird, die an der Schicht (3) adsorbiert wird.

16. Verfahren zur Herstellung einer Bioelektrode nach Anspruch 14 oder 15, **dadurch gekennzeichnet, dass** es außerdem einen Schritt k1) der Befestigung eines Drahtes aus einem elektrisch leitfähigen Material auf der Oberfläche der Schicht (2) aus Kohlenstoff-Nanoröhren, die nicht von der Schicht (3) aus aromatischen Molekülen bedeckt ist, umfasst, wobei dieser Schritt vor oder nach dem Schritt G) oder i1) der Abscheidung der Schicht (4) aus FAD-GDH-Enzymen durchgeführt wird.

17. Verfahren zur Herstellung einer Bioelektrode nach einem der Ansprüche 12 bis 16, **dadurch gekennzeichnet, dass** es weiterhin einen Schritt des Abscheidens einer Schicht (6) aus einem porösen und gegebenenfalls hydrophilen Material auf mindestens der freien Oberfläche der Schicht (4) umfasst.

18. Verfahren zur Herstellung einer Bioelektrode nach einem der Ansprüche 12 bis 17, **dadurch gekennzeichnet, dass** es weiterhin den Abscheidungsschritt zur Beschichtung der übereinanderliegenden Schicht (2) aus Kohlenstoff-Nanoröhren/Schicht (3) aus aromatischen Molekülen/Schicht (4) aus FAD-GDH-Enzymen/gegebenenfalls Träger (1)/Draht aus einem elektrisch leitfähigen Material, mit einem flüssigkeitsundurchlässigen Material, vorzugsweise auf Silikonbasis, umfasst.

## Claims

1. Bioelectrode, **characterized in that** it comprises the stacking of:
- a layer (2) of carbon nanotubes,
- a layer (3) which is placed on the layer (2) of carbon nanotubes and consists of aromatic molecules selected from the group consisting of 9,10-phenanthrenequinone, 1,10-phenanthroline-5,6-dione, 9,10-anthraquinone, phenanthrene, 1,10-phenanthroline, 5-methyl-1,10-phenanthroline, pyrene, 1-aminopyrene, pyrene-1-butyric acid and mixtures of two or more thereof,
- a layer (4) of flavin adenine dinucleotide-glucose dehydrogenase (FAD-GDH) enzymes adsorbed on the layer (3) of the aromatic molecules.

2. Bioelectrode according to claim 1, **characterized in that** it further comprises a layer (6) made of a porous and optionally hydrophilic material covering at least the free surface of the layer (4) of FAD-GDH enzymes, the layer (6) preferably being made of alginate, chitosan, Nafion® or silica gel.

3. Bioelectrode according to claim 1, **characterized in that** the layer (4) consists of 9,10-phenanthrenequinone or 1,10-phenanthroline-5,6-dione molecules.

4. Bioelectrode according to any of the preceding claims, **characterized in that** the layer (2) of carbon nanotubes comprises between 2.5 µg and 510 µg of carbon nanotubes per cm² of the surface of the layer, preferably between 2.5 and 100 µg of carbon nanotubes per cm² of the surface of the layer (2).

5. Bioelectrode according to any of the preceding claims, **characterized in that** it further comprises a substrate (1), which is made of an electron-conducting material, preferably made of glassy carbon, under the layer (2) of carbon nanotubes.

6. Bioelectrode according to any of claims 1 to 4, **characterized in that** it further comprises a wire made of an electrically conductive material connected to the surface of the layer (2) of carbon nanotubes which is not covered by the layer (3) of aromatic molecules.

7. Bioelectrode according to any of the preceding claims, **characterized in that** it further comprises a coating layer (5) which is impervious to fluids, which is preferably made of a silicone material, and which surrounds the stack of the layers (2), (3) and (4) and optionally the substrate 1.

8. Device comprising a bioelectrode according to any of claims 1 to 7.

9. Device according to claim 8, **characterized in that** it is an enzymatic biofuel cell.

10. Device comprising a bioelectrode according to any of claims 1 to 6, **characterized in that** it is a device for detecting glucose.

11. Use of a bioelectrode according to any of claims 1 to 6 or of a device according to claim 8 for oxidizing glucose.

12. Method for producing a bioelectrode according to any of claims 1 to 7, **characterized in that** it comprises the following steps:
a) preparing a suspension of carbon nanotubes having a concentration of carbon nanotubes of between 0.5 mg.mL⁻¹ and 10 mg.mL⁻¹, and preferably of 2.5 mg.mL⁻¹, in an organic solvent selected from among N-methyl-2-pyrrolidone (NMP), dichloromethane (DCM), acetonitrile (ACN), 1,3-dioxolane (DXL), dimethylformamide (DMF) and mixtures of two or more thereof, preferably NMP,
b) depositing a drop (drop-casting) of the suspension of carbon nanotubes obtained in step a) on a substrate (1) made of an electron-conducting material, preferably made of glassy carbon,
c) evaporating the organic solvent from the suspension of carbon nanotubes, whereby a layer (2) of carbon nanotubes is formed on the substrate (1),
d) preparing a solution comprising aromatic molecules selected from the group consisting of 9,10-phenanthrenequinone, 1,10-phenanthroline-5,6-dione, 9,10-anthraquinone, phenanthrene, 1,10-phenanthroline, 5-methyl-1,10-phenanthroline, pyrene, 1-aminopyrene, pyrene-1-butyric acid, and mixtures of two or more thereof, and having a concentration of between 0.5 mmol.L⁻¹ and 15 mmol.L⁻¹, and preferably 2.5 mmol.L⁻¹, of aromatic molecules dissolved in an organic solvent identical to or different from the organic solvent of the suspension of carbon nanotubes, the solvent being selected from among NMP, DCM, ACN, DXL, DMF and mixtures of two or more thereof, and preferably being NMP,
e) depositing, on a surface of the layer (2) of carbon nanotubes obtained in step c), the solution of aromatic molecules obtained in step d), by depositing a drop, preferably having a volume of between 5 and 200 µL, more preferably a volume of 175 µL, of the solution of aromatic molecules on the surface of the layer (2), or by incubating at least the free surface of the layer (2) in the solution of aromatic molecules obtained in step d),
f) evaporating the solvent from the solution of aromatic molecules deposited in step e), whereby a layer (3) of aromatic molecules is formed on the layer (2),
g) preparing an aqueous solution of FAD-GDH enzymes having a concentration of FAD-GDH enzymes of between 0.1 mg.mL⁻¹ and 5 mg.mL⁻¹, more preferably of 0.75 mg.mL⁻¹,
h) depositing, on the layer (3) of aromatic molecules obtained in step f), the solution of FAD-GDH enzymes obtained in step g), by incubating at least the free surface of the layer (3) of aromatic molecules in an aqueous solution containing the FAD-GDH enzymes obtained in step g), or by depositing a drop (drop-casting) of said aqueous solution containing the FAD-GDH enzymes obtained in step f) on the free surface of the layer (3).

13. Method for producing a bioelectrode according to claim 12, **characterized in that** step a) of depositing a drop of the dispersion of carbon nanotubes is repeated at least once.

14. Method for producing a bioelectrode according to any of claims 1 to 7, **characterized in that** it comprises the following steps:
A) preparing a suspension of carbon nanotubes in an organic solvent, preferably selected from among NMP, DCM, ACN, DXL, DMF and mixtures of two or more thereof, more preferably DMF, the suspension having a concentration of carbon nanotubes of between 1 mg.mL⁻¹ and 15 mg.mL⁻¹, preferably 1 mg.mL⁻¹,
B) preparing a solution of aromatic molecules selected from the group consisting of 9,10-phenanthrenequinone, 1,10-phenanthroline-5,6-dione, 9,10-anthraquinone, phenanthrene, 1,10-phenanthroline, 5-methyl-1,10-phenanthroline, pyrene, 1-aminopyrene, pyrene-1-butyric acid and mixtures of two or more thereof, preferably 1,10-phenanthroline-5,6-dione, in an organic solvent which is selected from among NMP, DCM, ACN, DXL, DMF and mixtures of two or more thereof and identical to or different from the organic solvent of the suspension of carbon nanotubes obtained in step a), preferably DMF, this solution of aromatic molecules having a concentration of aromatic molecules of between 0.5 mmol.L⁻¹ and 15 mmol.L⁻¹, preferably 2.5 mmol.L⁻¹,
C) adding the solution of aromatic molecules obtained in step b) into the suspension of nanotubes obtained in step A), and mixing,
D) filtering the dispersion obtained in step C) through a filtering substrate,
E) evaporating the solvent from the suspension of carbon nanotubes and from the solvent of the solution of aromatic mediator molecules, whereby a layer (2) of carbon nanotubes, which is itself covered on at least one of its surfaces by a layer (3) of said aromatic molecules, is formed,
F) preparing an aqueous solution of FAD-GDH enzymes having a concentration of FAD-GDH enzymes of between 0.1 and 5 mg.mL⁻¹,
G) depositing a drop of 150 µl of the solution of FAD-GDH enzymes prepared in step F) on the free surface of the layer (3), thus forming a layer (4) of adsorbed FAD-GDH enzymes on the layer (3) of aromatic molecules,
H) removing the assembly obtained in step G from the filtering substrate.

15. Method for producing a bioelectrode according to any of claims 1 to 7, **characterized in that** it comprises the following steps:
a1) preparing a suspension of carbon nanotubes in an organic solvent, preferably DMF, having a concentration of carbon nanotubes of between 1 mg.mL⁻¹ and 15 mg.mL⁻¹, preferably of 1 mg.mL⁻¹,
b1) preparing a solution of aromatic molecules selected from the group consisting of 9,10-phenanthrenequinone, 1,10-phenanthroline-5,6-dione, 9,10-anthraquinone, phenanthrene, 1,10-phenanthroline, 5-methyl-1,10-phenanthroline, pyrene, 1-aminopyrene, pyrene-1-butyric acid and mixtures of two or more thereof, preferably 1,10-phenanthroline-5,6-dione, in an organic solvent identical to or different from the organic solvent of the suspension of carbon nanotubes obtained in step a1), preferably DMF, this solution having a concentration of aromatic molecules of between 0.5 and 15 mmol.L⁻¹, preferably of 2.5 mmol.L⁻¹,
c1) filtering the dispersion obtained in step b1) through a filtering substrate (1),
d1) evaporating the solvent from the dispersion of carbon nanotubes deposited in step c), whereby a layer (2) of carbon nanotubes is formed,
e1) removing the layer of carbon nanotubes obtained in step d1) from the filtering substrate (1),
f1) depositing a drop of 150 µl of the solution of aromatic molecules obtained in step b1) on at least one surface of the layer (2) of carbon nanotubes obtained in step e1),
g1) evaporating the solvent from the solution of aromatic molecules deposited in step f1), whereby a layer (3) of said aromatic molecules is formed on the layer (2) of carbon nanotubes,
h1) preparing an aqueous solution of FAD-GDH enzymes having a concentration of FAD-GDH enzymes of between 0.1 and 5 mg.mL⁻¹,
i1) depositing a drop of 150 µl of the solution of FAD-GDH enzymes obtained in step h) on at least one surface of the layer (3) of aromatic molecules, thus forming a layer (4) of adsorbed FAD-GDH enzymes on the layer (3).

16. Method for producing a bioelectrode according to either claim 14 or claim 15, **characterized in that** it further comprises a step k1) of securing a wire made of an electrically conductive material on the surface of the layer (2) of carbon nanotubes not covered by the layer (3) of aromatic molecules, this step being implemented before or after step G) or i1) of depositing the layer (4) of FAD-GDH enzymes.

17. Method for producing a bioelectrode according to any of claims 12 to 16, **characterized in that** it further comprises a step of depositing a layer (6) made of a porous and optionally hydrophilic material on at least the free surface of the layer (4).

18. Method for producing a bioelectrode according to any of claims 12 to 17, **characterized in that** it further comprises a step of coating the stack of: the layer (2) of carbon nanotubes / the layer (3) of aromatic molecules / the layer (4) of FAD-GDH enzymes / optionally the substrate (1) / the wire made of an electrically conductive material, with a material impervious to fluids, preferably based on silicone.
